(19) Europäisches Patentamt
European Patent Office
Office européen des brevets

(11) EP 0 669 316 B1

(12) **FASCICULE DE BREVET EUROPEEN**

(45) Date de publication et mention de la délivrance du brevet: **30.07.1997 Bulletin 1997/31**

(51) Int. Cl.[6]: **C07C 279/12**, C07C 279/24, A61K 31/155

(21) Numéro de dépôt: **94403019.6**

(22) Date de dépôt: **26.12.1994**

(54) **Analogues de la 15-déoxyspergualine, leur procédé de préparation et leur utilisation en tant qu'agents immunosuppresseurs**

15-Deoxyspergualine-Analoge, Verfahren in ihrer Herstellung und ihre Verwendung als Immunosuppressiva

Analogs of 15-deoxyspergualine, process for their preparation and their use as immunosuppressive agents

(84) Etats contractants désignés:
**AT BE CH DE DK ES FR GB GR IE IT LI LU MC NL PT SE**

(30) Priorité: **24.02.1994 FR 9402125**
**01.06.1994 FR 9406706**

(43) Date de publication de la demande:
**30.08.1995 Bulletin 1995/35**

(73) Titulaire: **FOURNIER INDUSTRIE ET SANTE**
**F-75008 Paris (FR)**

(72) Inventeurs:
- **Renaut, Patrice**
  **F-21121 Hauteville-Lès-Dijon (FR)**
- **Lebreton, Luc**
  **F-21000 Dijon (FR)**
- **Dutartre, Patrick**
  **F-21110 Longchamp (FR)**
- **Derrepas, Philippe**
  **F-21410 Agey (FR)**
- **Samreth, Soth**
  **F-21600 Longvic (FR)**

(74) Mandataire: **Clisci, Serge et al**
**S.A. FEDIT-LORIOT & AUTRES**
**CONSEILS EN PROPRIETE INDUSTRIELLE**
**38, Avenue Hoche**
**75008 Paris (FR)**

(56) Documents cités:
**EP-A- 0 105 193** **EP-A- 0 181 592**

- **INT. J. IMMUNOPHARMAC., vol.14, no.4, 1992 pages 731 - 737 H. FUJI ET AL. 'Deoxyspergualin, a novel immunosuppressant, markedly inhibits human mixed lymphocyte reaction and cyctotoxic T-lymphocyte activity in vitro'**
- **JOURNAL OF MEDICINAL CHEMISTRY, vol.35, no.4, 1992, WASHINGTON US pages 724 - 734 J. R. LAKANEN ET AL. 'alpha-Methyl polyamines: metabolically stable spermidine and spermine mimics capable of supporting growth in cells depleted of polyamines'**
- **JOURNAL OF ANTIBIOTICS, vol.41, no.2, 1988, TOKYO JP pages 234 - 238 H. KARAMOCHI ET AL. 'The antiproliferative action of deoxyspergualin is different from that induced by amine oxidase'**
- **JOURNAL OF ANTIBIOTICS, vol.41, no.11, Novembre 1988, TOKYO JP pages 1629 - 1643 R. NISHIZAWA ET AL. 'Synthesis and biological activity of spergualin analogues'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 669 316 B1

Printed by Rank Xerox (UK) Business Services
2.14.11/3.4

## Description

### Domaine de l'invention

La présente invention concerne des nouveaux composés de structure apparentée à la 15-déoxyspergualine. Elle concerne également leur procédé de préparation et leur utilisation en thérapeutique, notamment en tant qu'agents immunosuppresseurs.

### Art Antérieur

On sait que la 15-déoxyspergualine, étudiée au départ pour son activité antitumorale, possède une bonne activité dans le domaine de l'immunosuppression. De nombreuses publications font état de cette activité, notamment : "Deoxyspergualin in lethal murine graftversus-host disease", Transplantation Vol 51, 712-715, No. 3, (Mars 1991) et "15-deoxyspergualin : From Cytostasis to Immunosuppression", Behring Inst. Mitt. No. 82, 231-239 (1988).

Cependant, la 15-déoxyspergualine ne présente pas une stabilité chimique satisfaisante et on a cherché à obtenir des dérivés plus stables, par exemple en remplaçant le résidu α-hydroxyglycine de la 15-déoxyspergualine par divers α-ou ω-aminoacides ou en modifiant le chaînon porteur de la fonction guanidine. Pour des exemples de telles modifications, on peut se référer à EP-A-0 181 592 ou à EP-A-0 105 193.

### Objet de l'invention

La présente invention propose de nouveaux produits, dont la structure générale reste apparentée à la 15-déoxyspergualine, qui sont chimiquement stables et qui présentent une activité immunosuppressive supérieure aux produits connus de l'art antérieur.

Les produits selon l'invention se distinguent notamment des produits connus de l'art antérieur du fait, dans leur structure chimique, de l'inversion de la liaison amide liant le reste guanidino-hexyle au chaînon central de la molécule, de la nature du chaînon central, et de l'introduction d'une chaîne ramifiée comportant un centre chiral dans la partie spermidine de la molécule.

Les composés analogues de la 15-déoxyspergualine selon l'invention sont caractérisés en ce qu'ils sont choisis parmi l'ensemble constitué par :

(i) les composés de formule :

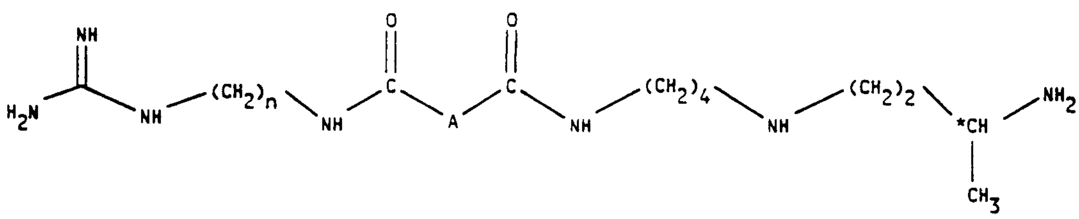

(I) ,

dans laquelle :

A représente une simple liaison, un groupe $-CH_2-$, un groupe $-CH_2O-$, un groupe $-CH_2NH-$, un groupe $-CH(OH)-$ un groupe $-CHF-$ ou un groupe $-CH(OCH_3)-$,
n est égal à 6 ou 8 ; et,

(ii) leurs sels d'addition.

Dans la formule I et les autres formules (i.e. II, IV, VI, XI, XI' et XII) qui suivent *C représente un atome de carbone asymétrique (autre nomenclature : "atome de carbone chiral").

Selon l'invention, on préconise également un procédé de préparation des composés de formule I et de leurs sels d'addition, ledit procédé comprenant la déprotection d'un composé de formule :

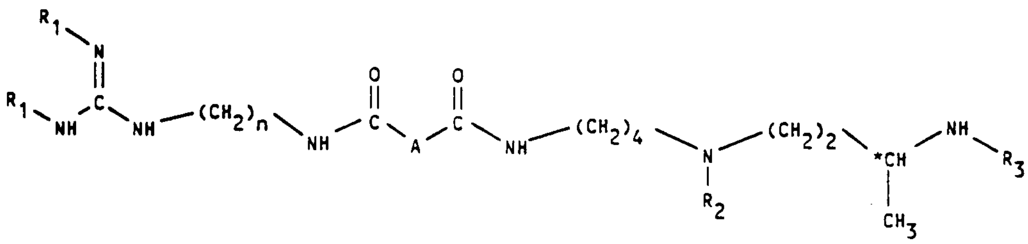

(II)

dans laquelle :

A et n sont définis comme indiqué ci-dessus, et
$R_1$, $R_2$ et $R_3$, identiques ou différents, représentent chacun un groupe protecteur de la fonction amine,
selon un ou plusieurs traitements réactionnels, connus de l'homme de l'art, pour obtenir le remplacement de tous les groupes $R_1$, $R_2$ et $R_3$ par un atome d'hydrogène.

On préconise également l'utilisation d'une substance choisie parmi les composés de formule I et leurs sels d'addition non toxiques, pour l'obtention d'un médicament destiné à une utilisation en thérapeutique vis-à-vis des désordres immunitaires, ou vis-à-vis du paludisme, ou encore en tant que réactif pharmacologique.

**Description détaillée de l'invention**

Par sels d'addition, on entend les sels d'addition d'acide obtenus par réaction d'un acide minéral ou d'un acide organique avec un composé de formule I. Les acides minéraux préférés pour la salification sont les acides chlorhydrique, bromhydrique, sulfurique et phosphorique. Les acides organiques préférés pour la salification sont les acides fumarique, maléique, méthanesulfonique, oxalique, citrique et trifluoroacétique.

Eu égard à la présence de l'atome de carbone asymétrique, noté *C ci-dessus, et à la nature du groupe A, les composés de formule I peuvent présenter un ou deux atomes de carbone chiraux. Quand A est $-CH_2-$, $-CH_2O-$ ou $-CH_2NH-$, la présente invention englobe, parmi les composés de formule I, les racémiques où *C est de configuration (R,S) et les énantiomères où *C est de configuration (R) ou (S). Quand A est -CH(OH)-, -CHF- ou $-CH(OCH_3)-$, la présente invention englobe, parmi les composés de formule I qui présentent alors deux sites chiraux, le mélange sensiblement équimoléculaire des quatre diastéréoisomères, les "hémiracémiques" (R,S)-A-(R)-*C, (R,S)-A-(S)-*C, (R)-A-(S,R)-*C et (S)-A-(S,R)-*C, et chacun des quatre diastéréoisomères.

De façon pratique, on préfère que l'atome de carbone asymétrique, noté *C, soit de configuration (R,S) ou (R).

Les composés de formule I peuvent être préparés selon des méthodes connues en soi, par application de mécanismes réactionnels classiques comme la formation d'une liaison amide, et notamment en appliquant les méthodes de la chimie des peptides.

Le procédé de préparation que l'on préconise suivant l'invention comprend, comme indiqué ci-dessus, la déprotection d'un composé de formule II.

De façon pratique, les groupes protecteurs $R_1$, $R_2$, $R_3$ que l'on va remplacer par un atome d'hydrogène seront des groupes amino-protecteurs de type connu dans le domaine de la chimie des peptides pour bloquer temporairement les fonctions "amine" non totalement substituées.

Parmi les groupes qui conviennent à cet effet, on peut utiliser :

(α) des groupes de type oxycarbonyle, comme par exemple les groupes alkoxycarbonyle et benzyloxycarbonyle :

Boc : t-butyloxycarbonyle [ou (1-1-diméthyléthoxy)carbonyle],
Fmoc : 9-fluorénylméthyloxycarbonyle,
Foc : furfuryloxycarbonyle,
Z : benzyloxycarbonyle,
Z(p-Cl) : 4-chlorobenzyloxycarbonyle, ou
Z(p-OMe) : 4-méthoxybenzyloxycarbonyle ;

(β) des groupes de type benzyle, comme par exemple :

Bn : phénylméthyle.

Parmi ces groupes amino-protecteurs, les groupes préférés sont les groupes Boc et Bn.

De façon pratique, le procédé de préparation d'un composé de formule I ou de l'un de ses sels d'addition est caractérisé en ce qu'il comporte les étapes consistant à :

**(i)** déprotéger un composé de formule II

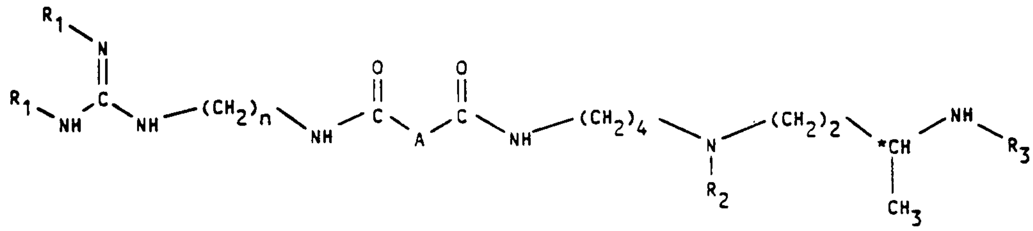

**(II)**

dans laquelle :

A représente une simple liaison, un groupe $CH_2$, un groupe CHF, un groupe $CH(OCH_3)$, un groupe CH(OH), un groupe $CH(OCH_2C_6H_5)$, un groupe $CH_2O$ ou un groupe $CH_2NH$, n est égal à 6 ou 8, et

$R_1$, $R_2$, $R_3$, identiques ou différents, représentent chacun un groupe amino-protecteur de type alkyloxycarbonyle, benzyloxycarbonyle ou benzyle,

selon un ou plusieurs traitements suivant la nature des groupes amino-protecteurs, par exemple, si l'un au moins des $R_1$, $R_2$ ou $R_3$ représente un groupe du type oxycarbonyle, par action d'un acide fort tel que notamment l'acide trifluoroacétique ou, si l'un au moins des $R_1$, $R_2$ ou $R_3$ représente un groupe du type benzyle ou si A représente le groupe $CH(OCH_2C_6H_5)$, par hydrogénation catalytique en présence de charbon palladié ou d'un sel de palladium, pour obtenir un composé de formule I sous forme de base libre ou de l'un de ses sels d'addition ; et,

**(ii)** si nécessaire, à partir d'un sel d'addition élaboré selon l'étape (i), obtenir le composé de formule I sous forme de base libre par action d'une base forte, puis obtenir à partir de ladite base libre les autres sels d'addition.

Pour la préparation d'un composé de formule II, on peut mettre en oeuvre l'une des variantes choisies parmi les suivantes :

**(a)** la variante A qui comprend l'étape consistant à : condenser un composé de formule :

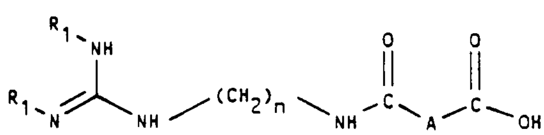

**(III)**

dans laquelle :

n est égal à 6 ou 8

A représente le groupe $CH_2$, $CH(OCH_3)$, $CH(OCH_2C_6H_5)$, CHF, ou une simple liaison,

$R_1$ représente un groupe amino-protecteur, tel que par exemple le groupe Boc,

sur une amine de formule :

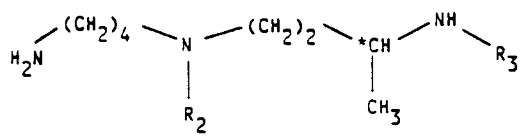

(IV)

dans laquelle :

$R_2$ et $R_3$, identiques ou différents, représentent chacun un groupe amino-protecteur, tel que par exemple les groupes Boc ou benzyle, (Bn),

par activation de l'acide :

- soit à l'aide d'un agent de couplage de type carbodiimide (notamment le 1,3-dicyclohexylcarbodiimide) en présence d'un agent nucléophile (comme par exemple le 1-hydroxybenzotriazole) dans un solvant organique, notamment un solvant chloré comme par exemple le chloroforme ou le dichlorométhane, et à une température comprise entre 0°C et 40°C,
- soit par formation d'un anhydride mixte à l'aide par exemple du chloroformiate d'isobutyle en présence d'un agent basique tel que notamment la N-méthylmorpholine, dans un solvant organique notamment le tétrahydrofuranne et à une température comprise entre -35°C et + 20°C,

à raison de 1 mole du composé III pour environ 1 mole du composé IV, pour obtenir un composé de formule:

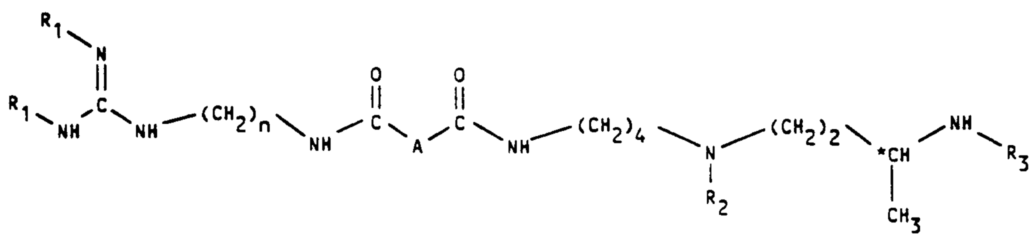

(II)

dans laquelle n, A, $R_1$, $R_2$ et $R_3$ ont les significations indiquées ci-dessus ;

**(b)** la variante B qui comprend les étapes consistant à :

**(i)** faire réagir un composé de formule :

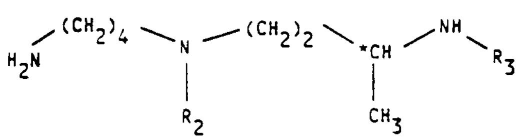

(IV)

dans laquelle :

$R_2$ et $R_3$, identiques ou différents, représentent chacun un groupe amino-protecteur de type oxycarbonyle ou benzyle,

avec un acide ou un chlorure d'acide de formule :

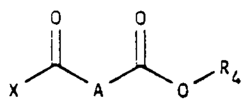

(V)

dans laquelle :

X représente un atome de chlore ou un groupe OH,
A représente une simple liaison, un groupe $CH_2$, un groupe CHF, un groupe $CH(OCH_3)$ ou un groupe $CH(OCH_2C_6H_5)$,
$R_4$ représente un groupe alkyle en $C_1$-$C_3$, linéaire ou ramifié, ou un groupe phénylméthyle,

dans un solvant organique, notamment un solvant chloré comme par exemple le dichlorométhane ou le chloroforme, en présence d'un activateur de groupe carboxylique comme par exemple un carbodiimide, (notamment le 1,3-dicyclohexylcarbodiimide ou le carbonyldiimidazole) et d'un agent nucléophile (notamment le 1-hydroxybenzotriazole) lorsque X représente le groupe OH, ou en présence d'une amine tertiaire (comme par exemple la triéthylamine) lorsque X représente un atome de chlore, à une température comprise entre 0°C et 40°C, à raison de 1 mole de IV pour environ 1 mole de V, pour obtenir un composé de formule :

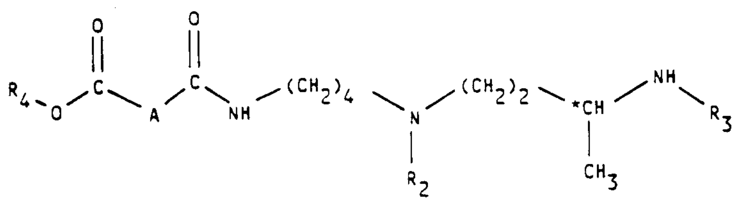

(VI)

dans laquelle A, $R_2$, $R_3$ et $R_4$ ont les significations indiquées ci-dessus ;
**(ii)** saponifier le composé de formule VI ainsi obtenu, dans un solvant organique, en présence d'une base forte pour obtenir un composé de formule :

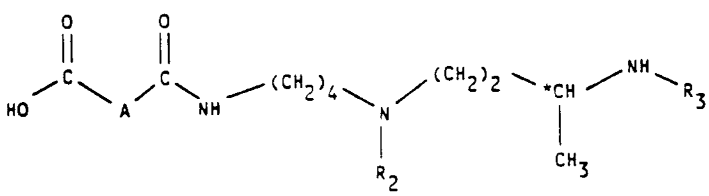

(VII)

dans laquelle A, $R_2$ et $R_3$ ont les significations indiquées ci-dessus ; et,
**(iii)** condenser le composé de formule VII ainsi obtenu, sur une amine de formule :

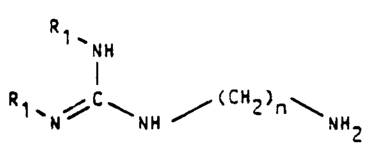

(VIII)

dans laquelle :

n est égal à 6 ou 8, et $R_1$ représente un groupe amino-protecteur comme par exemple un groupe Boc,

dans des conditions identiques à celles de la variante A ci-dessus, pour obtenir un composé de formule :

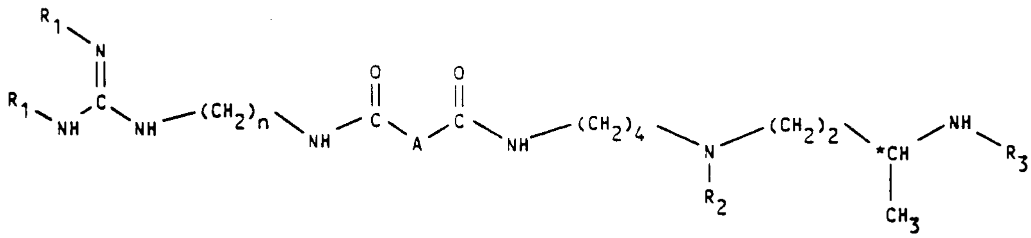

(II)

dans laquelle A, n, $R_1$, $R_2$ et $R_3$ ont les significations indiquées ci-dessus ;

**(c)** la variante C qui comprend les étapes consistant à :

**(i)** acyler l'extrémité $NH_2$ terminale d'une base de formule :

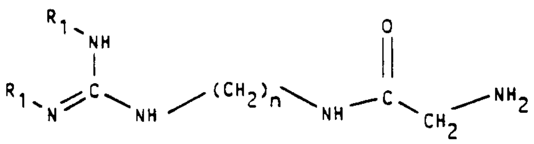

(IX)

dans laquelle $R_1$ représente un groupe amino-protecteur, notamment le groupe Boc, et n est égal à 6 ou 8, par un chloroformiate ou un carbonate symétrique [notamment le bis(4-nitrophényl)carbonate] dans un solvant inerte (comme par exemple le dichlorométhane), à température ambiante (15-25°C) ; et,
**(ii)** aminolyser le composé ainsi obtenu par une amine de formule :

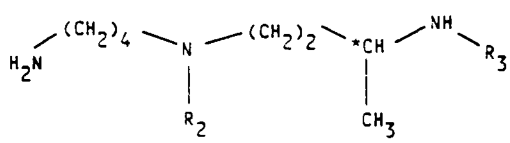

(IV)

dans laquelle :

$R_2$ et $R_3$, identiques ou différents, représentent chacun un groupe amino-protecteur notamment un groupe Boc ou phénylméthyle,

dans un solvant inerte notamment tel que le dichlorométhane, pour obtenir un composé de formule :

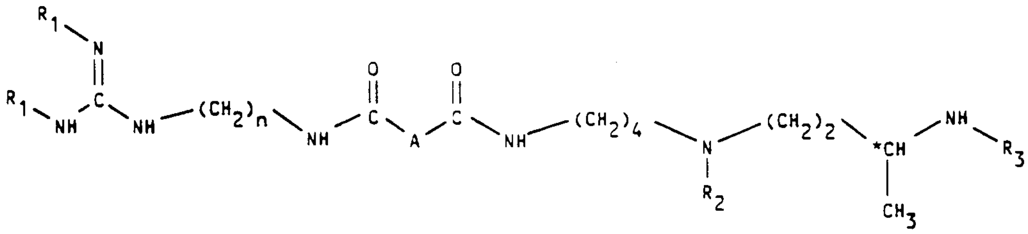

(II)

dans laquelle n, $R_1$, $R_2$ et $R_3$ ont les mêmes significations que ci-dessus et A représente le groupe $-CH_2-NH-$ ; et,

**(d)** la variante D qui comprend les étapes consistant à :

**(i)** faire réagir une amine de formule :

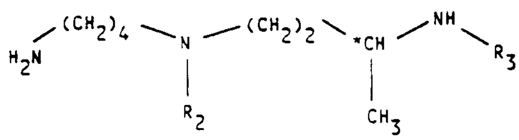

(IV)

dans laquelle :

$R_2$ et $R_3$, identiques ou différents représentent chacun un groupe amino-protecteur,

avec un carbonate de formule :

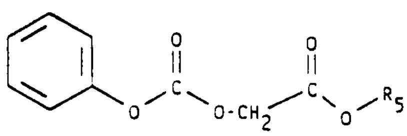

(X)

dans laquelle $R_5$ représente un groupe alkyle en $C_1$-$C_3$ ou un groupe phénylméthyle, dans un solvant inerte (notamment un solvant aromatique comme par exemple le toluène) à une température comprise entre la température ambiante et la température de reflux du milieu réactionnel, à raison de 1 mole de IV pour environ 1 mole de X, pour obtenir un composé de formule :

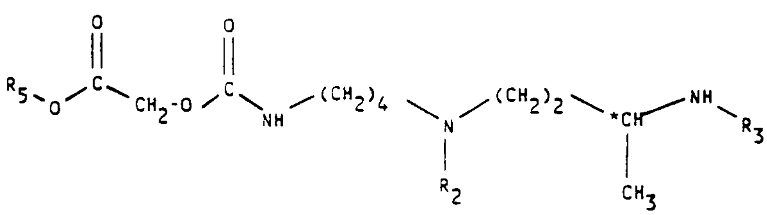

(XI)

dans laquelle $R_2$, $R_3$ et $R_5$ ont la même signification que ci-dessus, ou un composé de formule :

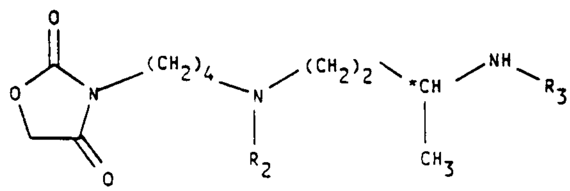

(XI')

dans laquelle $R_2$ et $R_3$ ont la même signification que ci-dessus ;

**(ii)** saponifier le composé XI ou XI' ainsi obtenu dans un solvant organique en présence d'une base forte pour obtenir un composé de formule :

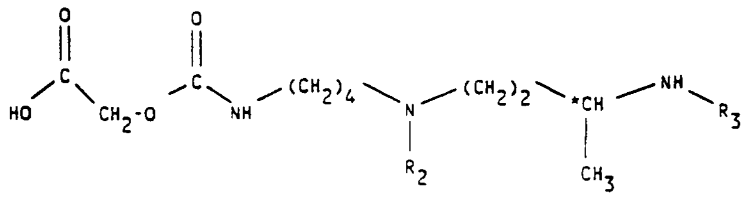

(XII)

dans laquelle $R_2$ et $R_3$ ont la même signification que ci-dessus : et,

**(iii)** condenser le composé de formule XII ainsi obtenu, sur une amine de formule :

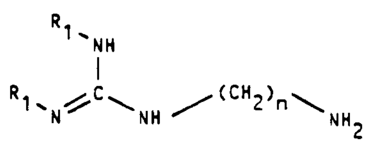

(VIII)

dans laquelle n est égal à 6 ou 8 et $R_1$ représente un groupe aminoprotecteur notamment le groupe Boc, dans des conditions identiques à celles décrites pour la variante A ci-dessus, pour obtenir un composé de formule :

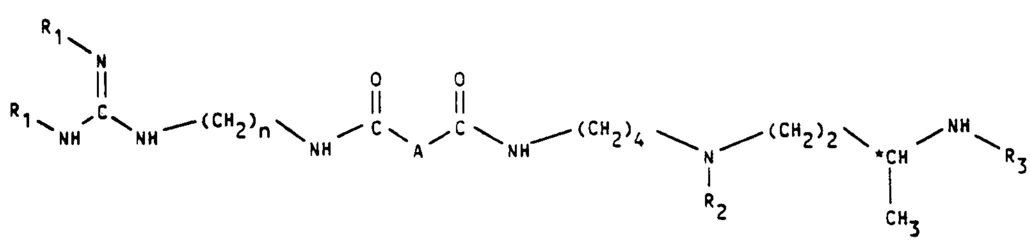

(II)

dans laquelle n, $R_1$, $R_2$ et $R_3$ ont les mêmes significations que ci-dessus, et A représente un groupe $CH_2O$.

L'invention sera mieux comprise à la lecture des exemples qui suivent et des résultats d'essais pharmacologiques obtenus avec les composés selon l'invention, comparativement aux résultats obtenus avec des produits connus de l'art antérieur. La nomenclature utilisée pour les exemples est celle préconisée par les Chemical Abstracts ; selon cette nomenclature un monoester d'un acide alcanedioïque avec le t-butanol est dénommé ici "acide alcanedioïque, (1,1-diméthyléthyl)

ester", et un diester du type alcanedioate de t-butyle et d'éthyle est dénommé ici "acide alcanedioïque, (1,1-diméthyléthyl) éthyl ester".

Dans la partie expérimentale, les préparations sont relatives aux intermédiaires et les exemples sont relatifs aux produits selon l'invention.

Lorsque les composés comportent dans leur structure un carbone asymétrique, l'absence d'indication particulière signifie qu'il s'agit d'un mélange sensiblement équimoléculaire des deux énantiomères (racémique).

Lorsque ces mêmes composés sont dénommés avec un signe (R) ou (S) à la suite immédiate de l'identification de la position d'un substituant, cela signifie que le carbone porteur de ce substituant est de configuration (R) ou (S), conformément aux règles de Cahn, Ingold et Prelog.

Lorsque les composés comportent dans leur structure deux centres d'asymétrie, l'absence d'indication particulière signifie qu'il s'agit du mélange des quatre diastéréoisomères.

Les caractéristiques spectrales des signaux de résonance magnétique nucléaire (RMN) sont données pour le proton ($^{1}H$) ou pour l'isotope 13 du carbone ($^{13}C$) : on indique le glissement chimique par rapport au signal du T.M.S., la forme du signal (s pour singulet, d pour doublet, t pour triplet, q pour quadruplet, m pour multiplet, sl pour signal large) et le nombre de protons concernés par le signal. A titre indicatif, les spectres RMN $^{1}H$ ont été réalisés à 300 MHz.

**PREPARATION 1**

**4-[N-(3-hydroxybutyl)-N-(phénylméthyl)-amino]-butanenitrile.**

On dissout 5 g ($28.10^{-3}$ mole) de 3-[N-(phénylméthyl)-amino]-1-méthylpropanol dans 60 ml de butanol et on ajoute 3,56 g ($34.10^{-3}$ mole) de carbonate de sodium, 1,06g ($7.10^{-3}$ mole) d'iodure de potassium puis 7,4 g ($70.10^{-3}$ mole) de 4-chlorobutyronitrile en solution dans 10 ml de butanol. On porte le mélange réactionnel sous agitation à reflux pendant 20 heures. Après refroidissement, on filtre et les matières insolubles sont rincées avec 60 ml d'éther éthylique. Les filtrats rassemblés sont concentrés sous pression réduite et le résidu résultant est repris par 100 ml de dichlorométhane.

La solution obtenue est extraite par 50 ml d'acide chlorhydrique 1 M ; la phase aqueuse acide obtenue est lavée 2 fois par 50 ml de dichlorométhane, puis rendue basique par addition lente de 50 ml d'une solution de soude 5N. Le produit est extrait de la phase aqueuse par 3 fois 50 ml de dichlorométhane. La phase organique obtenue est séchée sur carbonate de potassium puis concentrée sous pression réduite. Le liquide jaune obtenu (7 g) est ensuite distillé sous vide pour obtenir 6,02 g (Rendement = 87 %) du produit attendu.

Eb = 160-170° C / 0,05 mm Hg.
(0,05 mm Hg correspond à environ 0,066 Pa)

**PREPARATION 2**

**Acide méthanesulfonique, 3-[N-(3-cyanopropyl)-N-(phénylméthyl)amino]-1-méthylpropyl ester.**

On prépare une solution de 9,11 g ($37.10^{-3}$ mole) du produit obtenu selon la préparation 1 dans 150 ml d'éther éthylique anhydre et on refroidit à 0° C. On ajoute ensuite 11,23 g ($111.10^{-3}$ mole) de triéthylamine puis, goutte à goutte, 4,66 g ($40.10^{-3}$ mole) de chlorure de méthanesulfonyle. On agite à 0° C pendant 1 heure après la fin de l'addition, puis pendant 15 heures à température ambiante. On ajoute lentement 120 ml d'une solution saturée de bicarbonate de sodium, puis on extrait le milieu réactionnel avec 3 fois 50 ml d'éther éthylique. Les phases organiques sont rassemblées et séchées sur carbonate de potassium puis concentrées sous pression réduite. On obtient ainsi 11,7 g (Rendement = 98 % en produit brut) du produit attendu sous forme d'une huile visqueuse jaune. Le produit est utilisé sans purification complémentaire pour l'étape suivante mais peut-être obtenu pur par chromatographie sur silice et élution avec un mélange n-heptane/éther éthylique (7/3) v/v.

RMN $^{1}H$ ($CDCl_3$) : 1,33 (d,3H) ; 1,76 (m,3H) ; 1,86 (m,1H) ; 2,37 (t, 2H) ; 2,53 (m,4H) ; 2,89 (s,3H) ; 3,48 (d,1H) ; 3,57 (d,1H) ; 4,84 (m,1 H) ; 7,28 (m,5H).

**PREPARATION 3**

**3-[N-(3-cyanopropyl)-N-(phénylméthyl)-amino]-1-méthyl-1-azidopropane.**

On prépare une solution de 10,59 g ($33.10^{-3}$ mole) du produit de la préparation 2 dans 70 ml de diméthylsulfoxyde et on ajoute 6,43 g ($100.10^{-3}$ mole) d'azoture de sodium. Le mélange réactionnel est agité pendant 15 heures à 45-50°C puis refroidi ; on ajoute 100 ml d'eau puis on extrait avec 100 ml d'éther éthylique. La phase aqueuse décantée est à nouveau extraite 3 fois avec 30 ml d'éther éthylique et les phases organiques rassemblées sont lavées avec 50 ml d'une solution aqueuse saturée en chlorure de sodium, puis séchées sur sulfate de magnésium. Après élimination

du solvant sous pression réduite, le liquide résiduel est purifié par chromatographie sur silice et élution avec un mélange n-heptane/éther éthylique (7/3) v/v. On obtient ainsi 7,8 g (Rendement = 78 %) du produit attendu sous forme d'une huile visqueuse incolore.

RMN $^1H$ ($CDCl_3$) : 1,22 (d,3H) ; 1,58 (m,2H) ; 1,77 (m,2H) ; 2,34 (t,2H) ; 2,52 (m,4H) ; 3,50 (m,3H) ; 7,26 (m,5H).

**PREPARATION 4**

**Acide 9-cyano-3-méthyl-6-(phénylméthyl)-2,6-diazanonanoïque, 1,1-diméthyléthyl ester.**

On introduit dans un flacon à hydrogéner d'un volume de 250 ml, 3,00 g ($11.10^{-3}$ mole) du produit obtenu selon la préparation 3 et 2,85 g ($13.10^{-3}$ mode) de dicarbonate de di-tert.-butyle (produit de structure : $O[CO_2C(CH_3)_3]$) en solution dans 30 ml d'acétate d'éthyle anhydre, et on ajoute 0,3 g de charbon palladié à 10 %. Le mélange est hydrogéné sous agitation, à température ambiante et sous une pression de $2.10^5$ Pa pendant 15 heures. On élimine ensuite le catalyseur par filtration puis on concentre sous pression réduite.

Le produit résiduel est purifié par chromatographie sur gel de silice et élution avec un mélange n-heptane/éther éthylique (45/55) v/v. On obtient ainsi 3,47 g (Rendement = 91 %) du produit attendu sous forme d'une huile visqueuse incolore.

RMN $^1H$ ($CDCl_3$) : 1,06 (d,3H) ; 1,44 (s,9H) ; 1,45-1,80 (m,4H) ; 2,33 (t,1H) ; 2,48 (m,3H) ; 3,50 (q,2H) ; 3,70 (sl,1H) ; 5,08 (sl,1H) ; 7,27 (m,5H).

**PREPARATION 5**

**Acide 10-amino-3-méthyl-6-(phénylméthyl)-2,6-diazadécanoïque, 1,1-diméthyléthyl ester.**

On introduit dans un appareil à hydrogénation une suspension de 2 g de Nickel de Raney dans 180 ml d'éthanol anhydre. On sature cette suspension par barbottage d'ammoniac gazeux pendant 10 minutes puis on ajoute 2,95 g ($8.10^{-3}$ mode) du produit obtenu à la préparation 4 en solution dans 20 ml d'éthanol anhydre. On soumet ensuite le mélange réactionnel à une hydrogénation sous une pression de $10^6$ Pa pendant 15 heures à 40°C.

Après refroidissement, le catalyseur est éliminé par filtration et le filtrat est concentré sous pression réduite. L'huile résiduelle est purifiée par chromatographie sur gel de silice et élution avec un mélange méthanol/ammoniaque à 32 % (100/1) v/v. On obtient ainsi 2,7 g (Rendement = 91 %) du produit attendu sous forme d'une huile visqueuse incolore.

RMN $^1H$ ($CDCl_3$) : 1,04 (d,3H) ; 1,31 (s,2H) ; 1,44 (s,9H) ; 1,30-1,75 (m,6H) ; 2,63 (t,2H) ; 2,25-2,70 (m,4H) ; 3,43 (d,1H) ; 3,60 (d,1H) ; 3,60-3,75 (m,1H) ; 5,74 (sl,1H) ; 7,30 (m,5H).

**PREPARATION 6**

**Acide 3-[[(1,1-diméthyléthoxy)carbonyl]amino]-12-oxo-2,4,11-triazatétradéc-2-ènedioïque, 1-(1,1-diméthyléthyl) 14-éthyl ester.**

On dissout 1,05 g ($8.10^{-3}$ mole) d'acide malonique, éthyl ester (monoester) dans 15 ml de chloroforme anhydre puis on refroidit à 0°C et on ajoute 1,65 g ($8.10^{-3}$ mole) de 1,3-dicyclohexylcarbodiimide et 0,108 g ($0,8.10^{-3}$ mole) d'hydrate de 1-hydroxy-benzotriazole. Après avoir agité pendant 0,5 heure à 0°C, on ajoute une solution de 1,5 g ($4,19.10^{-3}$ mole) d'acide [[(6-aminohexyl)-imino]méthylène]biscarbamique, bis(1,1-diméthyléthyl) ester, en solution dans 5 ml de chloroforme anhydre. On laisse ensuite sous agitation pendant 48 heures, à température ambiante, puis on concentre le milieu réactionnel sous pression réduite. Le produit brut obtenu est purifié par chromatographie sur silice à moyenne pression et élution à l'aide d'un mélange hexane-acétate d'éthyle (1/1) v/v. On obtient ainsi 1,95 g (Rendement = 99 %) du produit attendu sous forme d'une huile jaune.

RMN $^1H$ ($CDCl_3$) : 1,30 (t,3H) ; 1,35-1,65 (m,26H) ; 3,2-3,35 (m,4H) ; 3,4 (td,2H) ; 4,2 (q,2H) ; 7,1-7,2 (sl,1H) ; 8,3 (t,1H) ; 11,5 (s,1H).

**PREPARATION 7**

**Acide 3-[[(1,1-diméthyléthoxy)carbonyl]amino]-12-oxo-2,4,11-triazatétradéc-2-ènedioïque, 1-(1,1-diméthyléthyl) ester.**

On dissout 1,95 g ($4,15.10^{-3}$ mole) du produit obtenu à la préparation 6 ci-dessus dans 15 ml de diméthoxyéthane et on ajoute 8,5 ml d'une solution aqueuse de NaOH 1N. On agite à température ambiante pendant 15 mn puis on ajoute 25 ml d'eau et 25 ml de chloroforme et on acidifie doucement jusqu'à pH = 2 à l'aide d'une solution aqueuse d'HCl 1N. On extrait plusieurs fois au chloroforme, puis on sèche la phase organique et concentre sous pression réduite. On obtient ainsi 1,8g (Rendement = 100%) du produit attendu sous forme d'une huile épaisse de couleur jaune.

RMN $^1H$ ($CDCl_3$) : 1,35-1,65 (m,26H) ; 3,3-3,45 (m,6H) ; 7,05 (sl,1H) ; 8,3 (t,1H) ; 9-11,5 (sl,2H).

**PREPARATION 8**

**Acide 3-[[(1,1-diméthyléthoxy)carbonyl]amino]-23-méthyl-12,14-dioxo-20-(phénylméthyl)-2,4,11,15,20,24-hexaazapentacos-2-ènedioïque, bis(1,1-diméthyléthyl) ester.**

En opérant de facon analogue à la préparation 6, mais au départ de 6,21 g ($14.10^{-3}$ mole) du produit obtenu à la préparation 7 et de 4,7 g ($13,5.10^{-3}$ mole) d'acide 10-amino-3-méthyl-6-(phényl-méthyl)-2,6-diazadécanoïque, 1,1 -diméthyléthyl ester, et après purification par chromatographie sur silice et élution avec un mélange acétate d'éthyle-éthanol (9/1) v/v, on obtient 8,5 g (Rendement = 81 %) du produit attendu sous forme d'huile orangée.

RMN 1H ($CDCl_3$) : 1,01-1,03 (d,3H) ; 1,23-1,70 (m,41H) ; 2,4-2,7 (m,4H) ; 3,05-3,3 (m,6H) ; 3,35-3,45 (td,2H) ; 3,6-3,8 (m,3H) ; 5,1-5,4 (sl,1H) ; 7,1-7,4 (m,5H) ; 7,55 (sl,1H) ; 7,75 (sl,1H) ; 8,3 (t,1H) ; 11,5 (s,1H).

**PREPARATION 9**

**Chlorhydrate de l'acide 3-[[(1,1-diméthyléthoxy)carbonyl]amino]-23-méthyl-12,14-dioxo-2,4,11,15,20,24-hexaazapentacos-2-ènedioïque, bis(1,1-diméthyléthyl) ester.**

On dissout 8,5 g ($10,9.10^{-3}$ mole) du produit obtenu selon la préparation 8 dans 100 ml d'éthanol et on ajoute 0,57 g de chlorure de palladium et 0,7 ml d'acide chlorhydrique concentré. Le mélange est ensuite hydrogéné à pression atmosphérique pendant 8 heures. On filtre le catalyseur, rince le catalyseur avec un peu d'éthanol, puis on concentre le filtrat résultant sous pression réduite. On obtient ainsi 7,8 g (Rendement = 98 %) du produit attendu sous forme d'une huile incolore.

RMN $^1H$ ($CDCl_3$) : 1,20 (d,3H) ; 1,25-1,9 (m,41H) ; 2,55-2,80 (m,2H) ; 2,80-3,55 (m,10H) ; 3,75 (sl,1H) ; 4,9 (d,1H) ; 7,9 (m,1H) ; 8,23 (sl,1H) ; 8,3 (t,1H) ; 11,5(s,1H).

**Exemple 1**

**N-[4-[[3-(amino)butyl]amino]butyl]-N'-[6-[(aminoiminométhyl)amino]-hexyl]propanediamide, tris(trifluoroacétate).**

On dissout 7,8 g ($10,8.10^{-3}$ mole) du produit obtenu suivant la préparation 9 précédente, dans 40 ml de dichlorométhane et on ajoute 40 ml d'acide trifluoroacétique. On agite le mélange réactionnel pendant 5 heures à température ambiante puis on concentre sous pression réduite. L'huile résiduelle est purifiée par chromatographie sur silice greffée RP18 (granulométrie 5-20 micromètres) à moyenne pression, et élution par un mélange eau/acétonitrile/acide trifluoroacétique (8/1/1) v/v. Les fractions pures ainsi obtenues sont rassemblées et lyophilisées. On reprend ensuite le lyophilisat avec 100 ml d'eau et on lave la solution avec deux fois 100 ml d'acétate d'éthyle, puis la phase aqueuse est à nouveau lyophilisée. On recommence deux fois cette opération pour éliminer l'acide trifluoroacétique. On obtient ainsi 4,5 g (Rendement = 57 %) du produit attendu sous forme d'un solide amorphe.

RMN $^1H$ (DMSO d6) : 1,15-1,20 (d,3H) ; 1,25-1,5 (m,8H) ; 1,5-1,85 (m,4H) ; 1,85-2,0 (m,2H) ; 2,85-3,2 (m,12H) ; 3,25-3,35 (m,1H) ; 6,8-7,55 (sl,3H) ; 7,6 (t,1H) ; 7,9-8,1 (m,5H) ; 8,5-8,75 (sl,3H). RMN $^{13}C$ ($D_2O$) : 18,01 ; 23,71 ; 26,21 ; 26,24 ; 26,35 ; 28,56 ; 28,84 ; 31,22 ; 39,51 ; 40,31 ; 41,88 ; 44,30 ; 44,60 ; 46,10 ; 48,14 ; 157,55 ; 170,01 ; 170,34.

**PREPARATION 10**

**Acide 3-méthyl-10-[2,4-dioxo-oxazolidine-3-yl]-6-(phénylméthyl)-2,6-diazadécanoïque, 1,1-diméthyléthyl ester.**

On prépare une solution de 3 g ($8,6.10^{-3}$ mole) d'acide 10-amino-3-méthyl-6-phénylméthyl-2,6-diazadécanoïque 1,1-diméthyléthyl ester dans 50 ml de toluène anhydre et on ajoute une solution de 1,8 g ($8,6.10^{-3}$ mole) d'acide phénoxycarbonyloxyacétique, méthyl ester dans 10 ml de toluène anhydre, puis 1,08 g ($10,7.10^{-3}$ mole) de triéthylamine. On porte à 60°C et on maintient à cette température sous agitation pendant 48 heures. On concentre ensuite le milieu réactionnel sous pression réduite et on purifie le résidu, ainsi obtenu par chromatographie sur silice et élution avec un mélange méthylcyclohexane/acétate d'éthyle (1/1) v/v. On obtient ainsi 3,1 g (Rendement = 82 %) de produit sous forme d'huile jaune visqueuse.

RMN $^1H$ ($CDCl_3$) : 1,0-1,1 (d,3H) ; 1,4-1,7 (m,15H) ; 2,35-2,50 (m,3H) ; 2,50-2,65 (m,1H) ; 3,40-3,60 (m,4H) ; 3,60-3,75 (m,1H) ; 4,65 (s,2H) ; 5,35 (sl,1H) ; 7,2-7,3 (m,5H).

**PREPARATION 11**

**Acide 3-méthyl-6-(phénylméthyl)-13-oxa-12-oxo-2,6,11-triazapentadécanedioïque, 1-(1,1-diméthyléthyl) ester.**

On dissout 3,1 g ($7,1.10^{-3}$ mole) du produit obtenu selon la préparation 10 ci-dessus dans 20 ml de diméthoxyéthane puis on ajoute 21 ml d'une solution aqueuse de 1N de NaOH. On agite à température ambiante pendant 4 heures puis on ajoute 75 ml d'eau et 75 ml de dichlorométhane. On acidifie jusqu'à pH = 2 à l'aide d'une solution 1N d'acide chlorhydrique et on extrait par du dichlorométhane. Les phases organiques sont lavées une fois avec une solution de chlorure de sodium, séchées sur sulfate de magnésium, puis concentrées sous pression réduite. On obtient ainsi 3 g (Rendement = 93 %) du produit attendu sous forme d'huile jaune épaisse.

RMN $^1H$ ($CDCl_3$) : 1,15 (d,3H) ; 1,2-2,2 (m,15H) ; 2,35-2,55 (t,2H) ; 2,7-2,9 (t,2H) ; 2,9-3,3 (m,4H) ; 3,5-3,75 (m,1H) ; 4,0-4,3 (m,2H) ; 4,9 (d,1H) ; 5,1 (d,1H) ; 7,4-7,8 (m,5H) ; 12,3 (sl,1H).

**PREPARATION 12**

**Acide 3-[[(1,1-diméthyléthoxy)carbonyl]amino]-24-méthyl-21-(phénylméthyl)-14-oxa-12,15-dioxo-2,4,11,16,21,25-hexaazahexacos-2-ènedioïque, bis(1,1-diméthyléthyl) ester.**

En opérant de façon analogue à la préparation 6, mais en utilisant le dichlorométhane comme solvant et au départ de 3 g ($6,65.\ 10^{-3}$ mole) de l'acide obtenu selon la préparation 11 et de 2,61 g ($6,65.10^{-3}$ mole) d'acide [[(6-aminohexyl)imino]méthylène]-biscarbamique, bis(1,1-diméthyléthyl) ester, on obtient, après purification par chromatographie sur silice et élution avec un mélange méthylcyclohexane/acétate d'éthyle (3/7) v/v, 4 g (Rendement = 76 %) du produit attendu sous forme d'huile jaune.

RMN $^1H$ ($CDCl_3$) : 0,9-1,1 (d,3H) ; 1,25-1,75 (m,41H) ; 2,3-2,5 (m,3H) ; 2,6 (m,1H) ; 3,1-3,2 (sl,2H) ; 3,2-3,35 (q,2H) ; 3,35-3,45 (t,2H) ; 3,5 (q,2H) ; 3,7 (m,1H) ; 4,5 (s,2H) ; 5,3-5,6 (sl,2H) ; 6,3 (51,1H) ; 7,2-7,3 (m,5H) ; 8,3 (t,1H) ; 11,5 (s,1H).

**PREPARATION 13**

**2-[[[4-[N'-[3-(amino)butyl]-N'-[phénylméthyl]-amino]butyl]amino]-carbonyloxy]-N-[6-[(aminoiminométhyl)amino]hexyl]-acétamide, tris(trifluoroacétate).**

En opérant de façon analogue à l'exemple 1 mais au départ de 4 g du produit obtenu selon la préparation 12 ci-dessus, et après purification par chromatographie sur colonne de silice greffée RP18 et élution avec un mélange eau/acétonitrile/acide trifluoroacétique (7,5/2/0,5) v/v, on obtient 4,2 g (Rendement = 99 %) du produit attendu sous forme d'huile épaisse.

RMN $^1H$ ($CDCl_3$) : 1,1-1,2 (d,3H) ; 1,2-1,6 (m,10H) ; 1,65-2,2 (m,4H) ; 2,9-3,2 (m,12H) ; 3,2-3,35 (m,1H) ; 4,35 (s,2H) ; 6,8-7,3 (sl,1H) ; 7,35 (t,1H) ; 7,45-7,65 (m,5H) ; 7,85-8,1 (m,3H) ; 9,8 (s,1H).

**Exemple 2**

**2-[[[4-[[3-(amino)butyl]amino]butyl]amino]carbonyloxy]-N-[6-[(amino-iminométhyl)amino]hexyl]-acétamide, tris(trifluoroacétate).**

On dissout 4,2 g ($5,05.10^{-3}$ mole) du produit obtenu à la préparation 13 ci-dessus dans 275 ml de méthanol, puis on ajoute 0,25 ml d'acide chlorhydrique concentré, puis 0,25 g de chlorure de palladium. Le mélange est ensuite hydrogéné à pression atmosphérique et à température ambiante pendant 14 heures. Le milieu réactionnel est filtré puis concentré sous pression réduite. L'huile obtenue est reprise par 100 ml d'eau et 1 ml d'acide trifluoroacétique, puis la solution est lavée avec 3 fois 75 ml d'acétate d'éthyle et lyophilisée. On obtient ainsi 3,9 g (Rendement = 99 %) du produit attendu sous forme d'un solide amorphe.

RMN $^1$H (DMSO d6) ; 1,2 (d,3H) ; 1,25-1,70 (m,12H) ; 1,70-1,9 (m,1H) ; 1,9-2,05 (m,1H) ; 2,85-3,15 (m,11H) ; 4,3 (s,2H) ; 6,8-7,5 (m,5H) ; 7,6-7,75 (t,1H) ; 7,85-7,95 (t,1H) ; 7,95-8,20 (sl,3H) ; 8,70-8,90 (sl,2H).
RMN $^{13}$C ($D_2O$) : 18,02 ; 23,65 ; 26,19 ; 26,28 ; 26,76 ; 28,56 ; 28,93 ; 31,22 ; 39,77 : 40,59 ; 41,88 ; 44,61 ; 46,11 ; 48,22 ; 63,76 ; 157,54 ; 158,21 ; 171,45.

**PREPARATION 14**

**Acide 3-[[(1,1-diméthyléthoxy)carbonyl]amino]-25-méthyl-22-phénylméthyl-14,16-dioxo-2,4,13,17,22,26-hexaazaheptacos-2-ènedioïque, bis(1,1-diméthyléthyl) ester.**

En procédant de façon identique à la préparation 6 mais au départ de 2,1g ($4,45.10^{-3}$ mole) d'acide 3-[[(1,1-diméthyléthoxy)carbonyl]amino]-14-oxo-2,4,13-triazahexadéc-2-ènedioïque, 1-(1,1-diméthyléthyl) ester et de 1,55 g ($4,45.10^{-3}$ mole) du composé obtenu à la préparation 5, on obtient après purification par chromatographie sur gel de silice et élution avec un mélange acétate d'éthyle/éthanol/ammoniaque (7/3/0,1) v/v, 3,26 g (Rendement = 93 %) du produit attendu sous forme de solide jaune amorphe.

RMN $^1$H ($CDCl_3$) : 1,02 (d,3H) ; 1,2-1,8 (m,45H) ; 2,55-2,8 (m,4H) ; 3,1-3,3 (m,6H) ; 3,38 (td,2H) ; 3,5-3,7 (m,1H) ; 3,7-3,9 (m,2H) ; 5-5,3 (sl,1H) ; 7,1-7,45 (m,5H) ; 7,5-7,6 (sl,1H) ; 7,7-7,8 (sl,1H) ; 8,3 (t,1H) ; 11,5 (s,1H).

**PREPARATION 15**

**Acide 3-[[(1,1-diméthyléthoxy)carbonyl]amino]-25-méthyl-14,16-dioxo-2,4,13,17,22,26-hexaazaheptacos-2-ènedioïque, bis-(1,1-diméthyléthyl) ester.**

On dissout 3,2 g ($4,06.\ 10^{-3}$ mole) du composé obtenu selon la préparation 14 dans 75 ml d'éthanol et on ajoute 0,25 g de charbon palladié à 5 %. On soumet le mélange à l'hydrogénation à température ambiante et à pression atmosphérique pendant 12 heures. On filtre le catalyseur et on concentre sous pression réduite. On obtient ainsi 2,15 g (Rendement = 74 %) du produit attendu sous forme d'un solide amorphe.

RMN $^1$H ($CDCl_3$) : 1,15 (d,3H) ; 1,25-1,8 (m,45H) ; 2,55-2,7 (m,4H) ; 3,11 (s,2H) ; 3,2-3,3 (m,4H) ; 3,39 (td,4H) ; 3,65-3,8 (m,1H) ; 4,8-4,9 (m,1H) ; 7,05 (sl, 1H) ; 7,55 (sl, 1H) ; 8,3 (t,1H) ; 11,5 (s,1H).

**Exemple 3**

**N-[4-[[3-(amino)butyl]amino]butyl]-N'-[8-[(aminoiminométhyl)amino]-octyl]-propanediamide, tris(trifluoroacétate).**

En opérant de façon analogue à l'exemple 1 mais au départ de 2,15 g ($3,01.10^{-3}$ mole) du composé obtenu selon la préparation 15, et après purification sur gel de silice RP18 et élution avec un mélange eau/acétonitrile/acide trifluoroacétique (7,5/2/0,5) v/v, on obtient 2,05 g (Rendement = 90 %) du produit attendu sous forme d'un solide amorphe.

RMN $^1$H (DMSO d6) : 1,18 (d,3H) ; 1,2-1,65 (m,16H) ; 1,65-1,8 (m,1H) ; 1,8-2 (m,1H) ; 2,8-3,1 (m,12H) ; 3,25-3,3 (m,1H) ; 6,8-7,5 (sl,3H) ; 7,55 (t,1H) ; 7,85-8,1 (m,5H) ; 8,45-8,65 (m,3H).
RMN $^{13}$C ($D_2O$) : 18,01 ; 23,69 ; 26,24 ; 26,51 ; 26,69 ; 28,60 ; 28,92 ; 28,96 ; 32,21 ; 39,49 ; 40,46 ; 41,97 ; 44,31 ; 44,59 ; 46,10 ; 48,14 ; 157,89 ; 169,96 ; 170,34.

**PREPARATION 16**

**Acide 3-[[(1,1-diméthyléthoxy)carbonyl]amino]-23-méthyl-20-(phénylméthyl)-13-(phénylméthoxy)-12,14-dioxo-2,4,11,15,20,24-hexaazapentacos-2-ènedioïque, bis(1,1 -diméthyléthyl) ester.**

On dissout 2,78 g ($5{,}05.10^{-3}$ mole) d'acide 3-[[(1,1-diméthyléthoxy)carbonyl]amino]-13-(phénylméthoxy)-12-oxo-2,4,11-triazatétradèc-2-ènedioïque, 1-(1,1 -diméthyléthyl) ester dans 50 ml de tétrahydrofuranne (THF) anhydre. On refroidit la solution à -25°C et on ajoute 1,02 g ($10{,}1.10^{-3}$ mole) de N-méthylmorpholine et 0,69 g ($5{,}05.10^{-3}$ mole) de chloroformiate d'isobutyle. Il se forme immédiatement un précipité blanc. On agite pendant 0,5 heure, puis on ajoute 1,76 g ($5{,}05.\ 10^{-3}$ mole) du composé obtenu selon la préparation 5, en solution dans 10 ml de THF. On agite pendant 1 heure puis on concentre sous pression réduite. Le produit est purifié par chromatographie sur gel de silice et élution avec un mélange méthylcyclohexane/acétate d'éthyle (7,5/2,5) v/v. On obtient ainsi 3,76 g (Rendement = 86 %) du produit attendu sous forme d'huile jaune.

RMN $^1H$ ($CDCl_3$) : 1,04 (dd,3H) ; 1,2-1,8 (m,41H) ; 2,3-2,7 (m,4H) ; 3,1-3,3 (m,4H) ; 3,3-3,5 (m,3H) ; 3,5-3,75 (m,2H) ; 4,28 (s,1H) ; 4,79 (s,2H) ; 5,3-5,5 (sl, 1H) ; 6,95 (sl, 1H) ; 7,2-7,4 (m, 10H) ; 8,3 (t, 1H) ; 11,5 (s, 1H).

**PREPARATION 17**

**Acide 3-[[(1,1-diméthyléthoxy)carbonyl]amino]-13-hydroxy-23-méthyl-12,14-dioxo-2,4,11,15,20,24-hexaazapentacos-2-ènedioïque, bis(1,1-diméthyléthyl) ester.**

En opérant de façon analogue à la préparation 15, mais au départ de 3,76 g ($4{,}35.10^{-3}$ mole) de produit obtenu selon la préparation 16, on obtient 3,05 g (Rendement quantitatif) du produit attendu sous forme d'huile jaune.

RMN $^1H$ ($CDCl_3$) : 1,15 (d,3H) ; 1,25-1,75 (m,42H) ; 2,55-2,7 (m,4H) ; 3,1-3,35 (m,4H) ; 3,35-3,45 (td,2H) ; 3,6-3,8 (m,1H) ; 4,42 (s,1H) ; 4,75-4,85 (m,1H) ; 7,2-7,45 (m,3H) ; 8,3 (t,1H) ; 11,5 (s,1H).

**Exemple 4**

**N-[4-[[3-(amino)butyl]amino]butyl]-N'-[6-[(aminoiminométhyl)amino]-hexyl]-2-hydroxy-propanediamide, tris(trifluoroacétate).**

En procédant de façon analogue à l'exemple 1, au départ de 3,03 g ($4{,}32.\ 10^{-3}$ mole) du composé obtenu selon la préparation 17, et après purification par chromatographie sur gel de silice RP18 et élution avec un mélange eau/acétonitrile/acide trifluoroacétique (8/1/1) v/v, on obtient 1,89 g (Rendement = 60 %) de solide amorphe blanc.

RMN $^1H$ (DMSO d6) : 1,18 (d,3H) ; 1,2-1,65 (m, 12H) ; 1,65-1,85 (m, 1H) ; 1,85-2 (m,1H) ; 2,8-3,15 (m,10H) ; 3,2-3,35 (m, 1H) ; 4,31 (s,1H) ; 6,7-7,4 (m,3H) ; 7,6 (t,1H) ; 7,85-8,05 (m,5H) ; 8,45-8,65 (m,3H).
RMN $^{13}C$ ($D_2O$) : 18,01 ; 23,66 ; 26,19 ; 26,28 ; 26,31 ; 28,55 ; 28,94 ; 31,21 ; 39,22 ; 40,00 ; 41,87 ; 44,59 ; 46,09 ; 48,12 ; 73,13 ; 157,23 ; 171,20 ; 171,53.

**PREPARATION 18**

**Acide 3-[[(1,1-diméthyléthoxy)carbonyl]amino]-15-phénylméthoxy-14-oxo-2,4,13-triazahexadèc-2-ènedioïque, 1-(1,1-diméthyléthyl) 16-éthyl ester.**

On dissout 2,6 g ($10{,}9.10^{-3}$ mole) d'acide 2-phénylméthoxypropanedioïque éthyl ester, dans 50 ml de dichlorométhane et on refroidit le mélange à 0°C. On ajoute 4,34 g ($22.10^{-3}$ mole) de N,N'-dicyclohexylcarbodiimide et 0,57 g ($4.10^{-3}$ mole) de 1-hydroxybenzotriazole et on maintient sous agitation pendant 0,5 heure, puis on ajoute 4,21 g (10,9. $10^{-3}$ mole) d'acide [[(8-amino-octyl)imino]méthylène]biscarbamique, bis(1,1-diméthyléthyl) ester en solution dans 15 ml de dichlorométhane et on laisse sous agitation à température ambiante pendant 48 heures. On concentre le milieu réactionnel sous pression réduite puis on purifie le résidu par chromatographie sur silice en éluant par un mélange méthylcyclohexane/acétate d'éthyle (7/3). On obtient ainsi 2,7 g (Rendement = 40,8 %) du produit attendu sous forme d'huile jaune pâle.

RMN $^1H$ ($CDCl_3$) : 1,25-1,6 (m,33H) ; 3,25 (td,2H) ; 3,39 (td,2H) ; 4,25 (q,2H) ; 4,44 (s,1H) ; 4,54 (d,1H) ; 4,70 (d,1H) ; 6,62 (t,1H) ; 7,3-7,45 (m,5H) ; 8,28 (t,1H) ; 11,5 (s,1H).

**PREPARATION 19**

**Acide 3-[[(1,1-diméthyléthoxy)carbonyl]amino]-15-phénylméthoxy-14-oxo-2,4, 13-triazahexadèc-2-ènedioïque, 1-(1,1-diméthyléthyl) ester.**

En opérant de façon analogue à la préparation 7 au départ de 2,7 g (4,45. $10^{-3}$ mole) du composé obtenu selon la préparation 18, on obtient 2,55 g (Rendement = 99 %) du produit attendu sous forme d'huile jaune.

RMN $^1H$ ($CDCl_3$) : 1,2-1,7 (m,30H) ; 3,2-3,4 (m,4H) ; 4,41 (s,1H) ; 4,71 (d,1H) ; 5,11 (d,1H) ; 6,99 (t,1H) ; 7,35-7,5 (m,5H) ; 8,3 (t,1H) ; 11,3-11,8 (sl,1H).

**PREPARATION 20**

**Acide 3-[[(1,1-diméthyléthoxy)carbonyl]amino]-15-phénylméthoxy-25-méthyl-22-phénylméthyl-14,16-dioxo-2,4,13,17,22,26-hexaazaheptacos-2-ènedioïque, bis-(1,1-diméthyléthyl) ester.**

En opérant de façon analogue à la préparation 16, au départ de 2,55 g (4,4.$10^{-3}$ mole) de produit obtenu selon la préparation 19 et de 1,54 g (4,4.$10^{-3}$ mole) du composé obtenu selon la préparation 5, on obtient 3,5 g (Rendement = 89 %) du produit attendu sous forme d'huile jaune.

RMN $^1H$ ($CDCl_3$) : 1,04 (dd,3H) ; 1,2-1,7 (m,45H) ; 2,3-2,65 (m,4H) ; 3,1-3,3 (m,4H) ; 3,39 (td,2H) ; 3,44 (d,1H) ; 3,57 (d,1H) ; 3,6-3,75 (m,1H) ; 4,28 (s,1H) ; 4,79 (s,2H) ; 5,35-5,5 (sl,1H) ; 6,85-7,1 (sl,2H) ; 7,2-7,4 (m,10H) ; 8,3 (t,1H) ; 11,5 (s,1H).

**PREPARATION 21**

**Acide 3-[[(1,1-diméthyléthoxy)carbonyl]amino]-15-hydroxy-25-méthyl-14,16-dioxo-2,4,13,17,22,26-hexaazaheptacos-2-ènedioïque, bis(1,1-diméthyléthyl) ester.**

En opérant de façon analogue à la préparation 15, au départ de 3,5 g (3,92.$10^{-3}$ mole) du composé obtenu selon la préparation 20, on obtient 2,4 g (Rendement = 85 %) du produit attendu sous forme d'huile jaune.

RMN $^1H$ ($CDCl_3$) : 1,15 (d,3H) ; 1,2-1,8 (m,46H) ; 2,55-2,75 (m,4H) ; 3,2-3,45 (m,6H) ; 3,65-3,8 (m,1H) ; 4,42 (s,1H) ; 4,75-4,9 (d,1H) ; 7,2 (t,1H) ; 7,4 (t,1H) ; 8,3 (t,1H) ; 11,5 (s,1H).

**Exemple 5**

**N-[4-[[3-(amino)butyl]amino]butyl]-2-hydroxy-N'-[8-[(aminoiminométhyl)amino]octyl]-propanediamide, tris(trifluoroacétate).**

En opérant de façon analogue au procédé de l'exemple 1, au départ de 2,4 g (3,29.$10^{-3}$ mole) du composé obtenu selon la préparation 21, on obtient après purification par chromatographie sur gel de silice RP18 en éluant avec un mélange eau/acétonitrile/acide trifluoroacétique (8/1,5/0,5), 2,27 g (Rendement = 89,5 %) du produit attendu sous forme d'un solide amorphe blanc.

RMN $^1H$ (DMSO d6) : 1,15-1,65 (m,19H) ; 1,65-1,85 (m,1H) ; 1,85-2 (m,1H) ; 2,85-3,2 (m,10H) ; 3,2-3,35 (m,1H) ; 4,31 (s,1H) ; 6,7-7,5 (sl,3H) ; 7,59 (t,1H) ; 7,8-8,05 (m,6H) ; 8,45-8,7 (m,2H).
RMN $^{13}C$ ($D_2O$) : 18,01 ; 23,65 ; 26,32 ; 26,49 ; 26,61 ; 28,60 ; 28,95 ; 29,04 ; 31,21 ; 39,20 ; 40,15 ; 41,97 ; 44,59 ; 46,09 ; 48,12 ; 73,14 ; 157,56 ; 171,15 ; 171,53.

**PREPARATION 22**

**Acide 3-[[(1,1-diméthyléthoxy)carbonyl]amino]-13-méthoxy-23-méthyl-20-phénylméthyl-12,14-dioxo-2,4,11,15,20,24-hexaazapentacos-2-ènedioïque, bis(1,1-diméthylèthyl) ester.**

En opérant de façon analogue au procédé de la préparation 16, au départ de 3 g (6,3. $10^{-3}$ mole) d'acide 3-[[(1,1-diméthyléthoxy)carbonyl]amino]-13-méthoxy-12-oxo-2,4,11-triazatétradèc-2-ènedioïque, 1-(1,1-diméthyléthyl) ester, et de 2,2 g.(6,3. $10^{-3}$ mole) du composé obtenu selon la préparation 5, on obtient 3,6 g (Rendement = 68 %) du produit attendu sous forme d'huile jaune.

RMN $^1H$ ($CDCl_3$) : 1,05 (dd,3H) ; 1,2-1,8 (m,41H) ; 2,3-2,7 (m,4H) ; 3,1-3,8 (m,12H) ; 4,1 (s,1H) ; 5,5 (m,1H) ; 6,9 (m,2H) ; 7,29 (m,5H) ; 8,29 (t,1H) ; 11,5 (s,1H).

**PREPARATION 23**

**Acide 3-[[(1,1-diméthyléthoxy)carbonyl]amino]-13-méthoxy-23-méthyl-12,14-dioxo-2,4,11,15,20,24-hexaazapentacos-2-ènedioïque, bis(1,1-diméthyléthyl) ester, chlorhydrate.**

On prépare une solution de 3,3 g ($4,1.10^{-3}$ mole) du composé obtenu selon la préparation 22 dans 100 ml d'éthanol et on ajoute 0,2 ml d'acide chlorhydrique concentré et 300 mg de charbon palladié à 10 %. On soumet le mélange à l'hydrogénation à température ambiante et à pression atmosphérique pendant 2 heures. On filtre le catalyseur et on concentre le filtrat sous pression réduite. On obtient ainsi 2,92 g (Rendement = 94,8 %) du produit attendu sous forme de solide blanc.

RMN $^1H$ ($CDCl_3$) : 1,1-2,3 (m,44H) ; 2,8-3,6 (m,11H) ; 3,4-3,6 (s,3H) ; 4,4 (s,1H) ; 4,9 (m,1H) ; 7,6 (m,1H) ; 7,9 (t,1H) ; 9,3 (m,1H) ; 9,9 (m,1H) ; 11,4 (s,1H).

**Exemple 6**

**N-[4-[[3-(amino)butyl]amino]butyl]-N'-[6-[(aminoiminométhyl)amino]-hexyl]-2-méthoxy-propanediamide, tris(trifluoroacétate).**

En opérant de façon analogue au procédé de l'exemple 1, au départ de 2,7 g ($3,6.10^{-3}$ mole) du composé obtenu selon la préparation 23, et après purification par chromatographie sur gel de silice RP18 et élution avec un mélange eau/acétonitrile/acide trifluoroacétique (8/1,5/0,5) v/v, on obtient 2,11 g (Rendement = 78 %) du produit attendu sous forme de solide amorphe.

RMN $^1H$ (DMSO d6) : 1,2 (d,3H) ; 1,4 (m,12H) ; 1,75-1,95 (2m,1H) ; 3,0 (m,11H) ; 3,3 (s,3H) ; 4,1 (s,1H) ; 7,1 (sl,3H) ; 7,6 (t,1H) ; 8.0 (m,4H) ; 8,6 (m,2H).
RMN $^{13}C$ ($D_2O$) : 18,01 ; 23,68 ; 26,18 ; 26,29 ; 26,32 ; 28,55 ; 28,92 ; 31,21 ; 39,21 ; 39,99 ; 41,87 ; 44,60 ; 46,10 ; 48,11 ; 58,65 ; 82,43 ; 157,54 ; 165,65 ; 169,96.

**PREPARATION 24**

**Acide 3-[[(1,1-diméthyléthoxy)carbonyl]amino]-15-méthoxy-14-oxo-2,4,13-triazahexadèc-2-ènedioïque, 1-(1,1-diméthyléthyl) 16-méthyl ester.**

En opérant de façon analogue au procédé de la préparation 18, au départ de 3,5 g ($23,65.10^{-3}$ mole) d'acide 2-méthoxy-propanedioïque, méthyl ester, et de 7 g ($18,1.10^{-3}$ mole) d'acide [[(8-aminooctyl)imino]méthylène]biscarbamique, bis(1,1-diméthyléthyl) ester, on obtient 8,6 g (Rendement = 95 %) du produit attendu sous forme d'huile jaune.

RMN $^1H$ ($CDCl_3$) : 1,2-1,65 (m,30H) ; 3,2-3,35 (m,2H) ; 3,38 (td,2H) ; 3,47 (s,3H) ; 3,82 (s,3H) ; 4,30 (s,1H) ; 6,6 (t,1H) ; 8,3 (t,1H) ; 11,5 (s,1H).

**PREPARATION 25**

**Acide 3-[[(1,1-diméthyléthoxy)carbonyl]amino]-15-méthoxy-14-oxo-2,4,13-triazahexadèc-2-ènedioïque, 1-(1,1-diméthyléthyl) ester.**

En opérant de façon analogue à la préparation 7, au départ de 8,6 g ($16,7.10^{-3}$ mole) du composé obtenu selon la préparation 24, on obtient 8,2 g (Rendement = 98 %) du produit attendu sous forme de solide amorphe jaune.

RMN $^1H$ ($CDCl_3$) : 1,25-1,7 (m,30H) ; 3,2-3,45 (m,4H) ; 3,69 (s,3H) ; 4,26 (s,1H) ; 6,9-7,1 (sl,1H) ; 8,25-8,45 (sl,1H) ; 11,3-11,9 (sl,1H).

**PREPARATION 26**

**Acide 3-[[(1,1-diméthyléthoxy)carbonyl]amino]-15-méthoxy-25-méthyl-22-phénylméthyl-14,16-dioxo-2,4,13,17,22,26-hexaazaheptacos-2-ènedioïque, bis(1,1-diméthyléthyl) ester.**

En opérant de façon analogue à la préparation 16, au départ de 4 g ($8.10^{-3}$ mole) du composé obtenu selon la préparation 25, on obtient 4,8 g (Rendement = 72 %) du produit attendu sous forme d'huile jaune clair.

RMN $^1H$ ($CDCl_3$) : 1,05 (dd,3H) ; 1,2-1,8 (m,45H) ; 2,3-2,7 (m,4H) ; 3,1-3,3 (m,4H) ; 3,3-3,6 (m,7H) ; 3,6-3,75 (m,1H) ; 4,09 (s,1H) ; 5,35-5,55 (sl,1H) ; 6,75-7 (m,2H) ; 7,2-7,35 (m,5H) ; 8,3 (t,1H) ; 11,5 (s,1H).

**PREPARATION 27**

**Acide 3-[[(1,1-diméthyléthoxy)carbonyl]amino]-15-méthoxy-25-méthyl-14,16-dioxo-2,4,13,17,22,26-hexaazaheptacos-2-ènedioïque, bis(1,1-diméthyléthyl) ester.**

En procédant de façon analogue à la préparation 23, au départ de 4,8 g ($5,76.10^{-3}$ mole) du composé obtenu selon la préparation 26, on obtient 4,15 g (Rendement = 97 %) du produit attendu sous forme d'huile.

RMN $^1H$ ($CDCl_3$) : 1,15 (d,3H) ; 1,25-1,8 (m,45H) ; 1,9-2,1 (sl,1H) ; 2,6-2,75 (m,4H) ; 3,2-3,35 (m,4H) ; 3,35-3,45 (m,2H) ; 3,57 (s,3H) ; 3,65-3,8 (m,1H) ; 4,12 (s,1H) ; 4,8-4,9 (sl,1H) ; 6,85 (t,1H) ; 7,2-7,35 (m,1H) ; 8,3 (t,1H) ; 11,5 (s,1H).

**Exemple 7**

**N-[4-[[3-(amino)butyl]amino]butyl]-2-méthoxy-N'-[8-[(aminoiminométhyl)amino]octyl]-propanediamide, tris(trifluoroacétate).**

En opérant de façon analogue au procédé de l'exemple 1, au départ de 4,15 g ($5,58.10^{-3}$ mole) du composé obtenu selon la préparation 27, on obtient 3,8 g (Rendement = 86,5 %) du produit attendu sous forme de solide blanc amorphe.

RMN $^1H$ (DMSO d6) : 1,18 (d,3H) ; 1,2-1,35 (m,8H) ; 1,35-1,65 (m,8H) ; 1,65-1,85 (m,1H) ; 1,85-2 (m,1H) ; 2,8-3,2 (m,10H) ; 3,25-3,4 (m,4H) ; 4,08 (s,1H) ; 6,6-7,5 (sl,3H) ; 7,62 (t,1H) ; 7,9-8,1 (m,6H) ; 8,5-8,7 (sl,2H).
RMN$^{13}C$ ($D_2O$) : 18,01 ; 23,67 ; 26,33 ; 26,50 ; 26,63 ; 28,60 ; 28,94 ; 28,93 ; 29,01 ; 31,21 ; 39,18 ; 40,14 ; 41,97 ; 44,59 ; 46,09 ; 48,11 ; 58,68 ; 82,44 ; 157,54 ; 169,60 ; 169,97.

**PREPARATION 28**

**Acide 13-fluoro-6-phénylméthyl-12-oxo-2,6,11-triazatétradécanedioïque, 1-(1,1-diméthyléthyl) 14-éthyl ester.**

On prépare une solution de 1,3 g ($8,7.10^{-3}$ mole) d'acide 2-fluoropropanedioïque, éthyl ester, dans 30 ml de dichlorométhane et 3 ml de diméthylformamide et on ajoute 2,2 g ($13,5.10^{-3}$ mole) de 1,1-carbonyldiimidazole. On agite pendant 3 heures à température ambiante puis on ajoute 3 g ($8,7.10^{-3}$ mole) du composé obtenu selon la préparation 5, en solution dans 10 ml de dichlorométhane. On maintient sous agitation pendant 48 heures à température ambiante. On concentre le milieu réactionnel sous pression réduite et purifie par chromatographie sur gel de silice et élution avec un mélange acétate d'éthyle/cyclohexane (6/4) v/v. On obtient ainsi 2,3 g (Rendement = 55,6 %) du produit attendu sous forme d'huile jaune.

RMN $^1H$ ($CDCl_3$) : 1,04 (d,3H) ; 1,2-1,7 (m,18H) ; 2,3-2,5 (m,2H) ; 2,5-2,65 (m,2H) ; 3,4-3,8 (m,5H) ; 4,25-4,4 (m,2H) ; 5,25 (d,2H) ; 6,7 (sl,1H) ; 7,3 (m,5H).

**PREPARATION 29**

**Acide 13-fluoro-6-phénylméthyl-12-oxo-2,6,11-triazatétradécanedioïque, 1-(1,1-diméthyléthyl) ester.**

En opérant de façon analogue à la préparation 7, au départ de 2,25 g ($4,7.10^{-3}$ mole) du produit obtenu selon la préparation 28, on obtient 1,44 g (Rendement = 68 %) du produit attendu sous forme d'une poudre blanche.

**F = 50° C**

RMN $^1H$ ($CDCl_3$) : 1,1 (d,3H) ; 1,4-2,1 (m,15H) ; 2,7-3,5 (m,7H) ; 4,0-4,3 (m,2H) ; 4,8 (m,1H) ; 5,4 (m,1H) ; 6,3-6,8 (m,1H) ; 7,4 (m,5H).

**PREPARATION 30**

**Acide 3-[[(1,1-diméthyléthoxy)carbonyl]amino]-13-fluoro-23-méthyl-20-phénylméthyl-12,14-dioxo-2,4,11,15,20,24-hexaazapentacos-2-ènedioïque, bis(1,1-diméthyléthyl) ester.**

En opérant de façon analogue au procédé de la préparation 16, au départ de 670 mg ($1,5.10^{-3}$ mole) du composé obtenu selon la préparation 29 et de 530 mg ($1,5.10^{-3}$ mole) d'acide [[6-amino-hexyl)imino]méthylène]biscarbamique, bis(1,1-diméthyléthyl) ester, on obtient 570 mg (Rendement = 49 %) du produit attendu sous forme d'une huile jaune clair.

RMN $^1H$ ($CDCl_3$) : 1,0-1,1 (d,3H) ; 1,2-1,7 (m,41H) ; 2,3-2,7 (m,4H) ; 3,2-3,8 (m,9H) ; 5,07-5,2 (dd,1H) ; 6,8-7,1 (m,2H) ; 7,2-7,4 (m,5H) ; 8,29 (t,1H) ; 11,5 (s,1H).

**PREPARATION 31**

**Acide 3-[[(1,1-diméthyléthoxy)carbonyl]amino]-13-fluoro-23-méthyl-12,14-dioxo-2,4,11,15,20,24-hexaazapentacos-2-énedioïque, bis(1,1-diméthyléthyl) ester, chlorhydrate.**

En opérant de façon analogue au procédé de la préparation 23, au départ de 560 mg ($0,7.10^{-3}$ mole) du produit obtenu selon la préparation 30, on obtient 484 mg (Rendement = 93 %) du produit attendu sous forme de solide blanc.

RMN $^1H$ ($CDCl_3$) : 1,1-2,1 (m,44H) ; 3,0-3,8 (m,11H) ; 4,8 (m,1H) ; 5,4-5,6 (d,1H) ; 8 (m,1H) ; 8,5 (m,1H) ; 9,7 (m,1H) ; 10,7 (m,1H) ; 11,3 (s,1H).

**Exemple 8**

**N-[4-[[3-(amino)butyl]amino]butyl]-2-fluoro-N'-[6-[(aminoiminométhyl)-amino]hexyl]-propanediamide, tris(trifluoroacétate).**

En procédant de façon analogue au procédé de l'exemple 1, au départ de 460 mg ($0,62.10^{-3}$ mole) du composé obtenu selon la préparation 31, on obtient 350 mg (Rendement = 67 %) du produit attendu sous forme d'un solide amorphe blanc.

RMN $^1H$ (DMSO d6) : 1,1 (d,3H) ; 1,2-1,6 (m,12H) ; 1,7-1,9 (2m,2H) ; 2,8-3,2 (m,10H) ; 3,27 (m,1H) ; 5,1-5,28 (d,1H) ; 7,1 (m,4H) ; 7,6 (t,1H) ; 7,95 (s,3H) ; 8,35-8,65 (m,4H).
RMN $^{13}C$ (DMSO d6) : 17,89 ; 22,83 ; 25,67 ; 25,81 ; 25,86 ; 28,34 ; 28,68 ; 30,40 ; 37,87 ; 38,45 ; 40,64 ; 43,17 ; 44,38 ; 46,43 ; 86,46 ; 156,51 ; 170,12 ; 170,14.

**PREPARATION 32**

**Acide 3-[[(1,1-diméthyléthoxy)carbonyl]amino]-15-fluoro-25-méthyl-22-phénylméthyl-14,16-dioxo-2,4,13,17,22,26-hexaazaheptacos-2-ènedioïque, bis(1,1-diméthyléthyl) ester.**

En opérant de façon analogue à la préparation 16, au départ de 670 mg ($1,5.10^{-3}$ mole) du composé obtenu selon la préparation 29 et de 571 mg ($1,5.10^{-3}$ mole) d'acide [[(8-aminooctyl)imino]méthylène]biscarbamique, bis( 1,1-diméthyléthyl) ester, on obtient 1 g (Rendement = 83.3 %) du produit attendu sous forme d'huile jaune.

RMN $^1H$ ($CDCl_3$) : 1,06 (d,3H) ; 1,2-1,8 (m,45H) ; 2,5 (m,4H) ; 3,2-3,8 (m,9H) ; 5,1-5,2 (d,1H) ; 5,2 (m,1H) ; 6,9 (m,2H) ; 7,3 (m,5H) ; 8,23 (t,1H) ; 11,5 (s,1H).

**PREPARATION 33**

**Acide 3-[[(1,1-diméthyléthoxy)carbonyl]amino]-15-fluoro-25-méthyl-14,16-dioxo-2,4,13,17,22,26-hexaazaheptacos-2-ènedioïque, bis(1,1-diméthyléthyl) ester, chlorhydrate.**

En opérant de façon analogue à la préparation 23, au départ de 1 g ($1,2.10^{-3}$ mole) du composé obtenu selon la

préparation 32 on obtient 746 mg (Rendement = 81 %) du produit attendu sous forme de solide blanc hygroscopique.

RMN $^1$H ($CDCl_3$) : 1,1-2,1 (m,48H) ; 2,8-3,5 (m,9H) ; 3,6-3,8 (m,2H) ; 4,9 (sl,1H) ; 5,6 (d,2H) ; 7,9 (sl,1H) ; 8,5 (m,1H) ; 11,4 (m,1H).

**Exemple 9**

**N-[4-[[3-(amino)butyl]amino]butyl]-2-fluoro-N'[8-[(aminoiminométhyl)-amino]octyl]-propanediamide, tris(trifluoroacétate).**

En opérant de façon analogue au procédé de l'exemple 1, au départ de 740 mg ($0,96.10^{-3}$ mole) du composé obtenu selon la préparation 33, on obtient 400 mg (Rendement = 54 %) du produit attendu sous forme d'un solide blanc amorphe.

RMN $^1$H (DMSO d6) : 1,1-1,6 (m,19H) ; 1,75-1,95 (2m,2H) ; 2,8-3,2 (m,10H) ; 3,3 (m,1H) ; 5,12-5,28 (d,1H) ; 7,1 (sl,1H) ; 7,6 (t,1H) ; 7,94 (s,3H) ; 8,3-8,6 (m,4H).
RMN $^{13}$C ($D_2O$) : 18,01 ; 23,65 ; 26,19 ; 26,48 ; 26,57 ; 28,59 ; 28,88 ; 28,91 ; 28,94 ; 31,21 ; 39,30 ; 40,22 ; 41,97 ; 44,59 ; 46,10 ; 48,09 ; 87,27 ; 89,87 ; 156,81 ; 170,12 ; 170,14.

**PREPARATION 34**

**Acide 3-méthyl-6-phénylméthyl-12-oxo-2,6,11-triazatridécanedioïque, 1-(1,1-diméthyléthyl) 13-éthyl ester.**

On dissout 7 g ($20.10^{-3}$ mole) du composé obtenu selon la préparation 5 dans 100 ml de dichlorométhane anhydre et on ajoute 5,25 g ($52.10^{-3}$ mole) de triéthylamine, puis lentement 3,56 g ($26.10^{-3}$ mole) de chlorure d'éthoxalyle en refroidissant au bain d'eau à 10° C. On agite 15 mn après la fin de l'addition puis on concentre sous pression réduite. On purifie par chromatographie sur gel de silice et élution avec un mélange méthylcyclohexane/acétate d'éthyle (4/6 puis 1/9) v/v. On obtient ainsi 6,8 g (Rendement = 75,7 %) du produit attendu sous forme d'huile jaune.

RMN $^1$H ($CDCl_3$) : 1,0 (d,3H) ; 1,3-1,8 (m,18H) ; 2,3-2,65 (m,4H) ; 3,2-3,35 (m,2H) ; 3,4 (d,1H) ; 3,58 (d,1H) ; 3,6-3,8 (m,1H) ; 4,3 (q,2H) ; 5,2-5,4 (sl,1H) ; 7,2-7,5 (m,6H).

**PREPARATION 35**

**Acide 3-méthyl-6-phénylméthyl-12-oxo-2,6,11-triazatridécanedioïque, 1-(1,1-diméthyléthyl) ester.**

En opérant de façon analogue à la préparation 7, au départ de 6,8 g ($15,14.10^{-3}$ mole) du composé obtenu selon la préparation 34, on obtient 6,4 g (Rendement quantitatif) du produit attendu sous forme d'un solide amorphe blanc.

RMN $^1$H ($CDCl_3$) : 1,1 (d,3H) ; 1,3-1,9 (m,15H) ; 2,5-3,45 (m,8H) ; 3,5-3,7 (m,1H) ; 5,3-5,6 (sl,1H) ; 7,25-7,6 (m,5H) ; 7,7-8 (sl,1H).

**PREPARATION 36**

**Acide 3-[[(1,1-diméthyléthoxy)carbonyl]amino]-22-méthyl-19-phénylméthyl-12,13-dioxo-2,4,11,14,19,23-hexaazatétracos-2-énedioïque, bis(1,1-diméthyléthyl) ester.**

En opérant de façon analogue à la préparation 18, au départ de 4 g ($9,5.10^{-3}$ mole) du composé obtenu selon la préparation 35 et de 3,4 g ($9,5.10^{-3}$ mole) d'acide [[(6-aminohexyl)imino]méthylène]biscarbamique, bis(1,1-diméthyléthyl) ester, on obtient 1,46 g (Rendement = 20 %) du produit attendu sous forme d'huile jaune.

RMN $^1$H ($CDCl_3$) : 1,05 (d,3H) ; 1,3-1,8 (m,41H) ; 2,3-2,6 (m,4H) ; 3,1-3,35 (m,4H) ; 3,40 (td,1H) ; 3,45 (d,1H) ; 3,6 (d,1H) ; 3,6-3,8 (m,1H) ; 5,3-5,5 (sl,1H) ; 7,2-7,35 (m,5H) ; 7,4-7,6 (m,2H) ; 8,3 (t,1H) ; 11,5 (s,1H).

**PREPARATION 37**

**Acide 3-[[(1,1-diméthyléthoxy)carbonyl]amino]-22-méthyl-12,13-dioxo-2,4,11,14,19,23-hexaazatétracos-2-énedioïque, bis(1,1-diméthylèthyl) ester.**

En opérant de façon analogue à la préparation 15, au départ de 1,46 g du composé obtenu selon la préparation 36, on obtient 1,25 g (Rendement = 97,5 %) du produit attendu sous forme d'huile jaune.

RMN $^1$H ($CDCl_3$) : 1,2 (d,3H) ; 1,3-1,8 (m,41H) ; 2,6-2,8 (m,4H) ; 3,25-3,85 (m,8H) ; 4,85 (d,1H) ; 7,45 (t,1H) ; 7,85 (t,1H) ; 8,3 (t,1H) ; 11,5 (s,1H).

**Exemple 10**

**N-[4-[[3-(amino)butyl]amino]butyl]-N'-[6-[(aminoiminométhyl)amino]-hexyl]-éthanediamide, tris(trifluoroacétate).**

En opérant de façon analogue au procédé de l'exemple 1, au départ de 1,25 g du composé obtenu selon la préparation 37, on obtient 580 mg (Rendement = 43 %) du produit attendu sous forme d'un solide amorphe blanc.

RMN $^1$H (DMSO $d_6$) : 1,2 (d,3H) ; 1,25-1,65 (m,12H) ; 1,65-1,85 (m,1H) ; 1,85-2,0 (m,1H) ; 2,8-3,2 (m,10H) ; 3,2-3,4 (m,1H) ; 6,8-7,5 (sl,3H) ; 7,65 (t,1H) ; 7,9-8,1 (sl,4H) ; 8,5-8,7 (sl,2H) ; 8,71 (t,1H) ; 8,77 (t,1H).
RMN $^{13}$C ($D_2O$): 18,00 ; 23,77 ; 26,13 ; 26,23 ; 26,41 ; 28,54 ; 28,82 ; 31,21 ; 39,54 ; 40,31 ; 41,88 ; 44,61 ; 46,09 ; 48,12 ; 157,55 ; 161,65 ; 161,98.

**PREPARATION 38**

**Acide 3-[[(1,1-diméthyléthoxy)carbonyl]amino]-24-méthyl-21-phénylméthyl-14,15-dioxo-2,4,13,16,21,25-hexaazahexacos-2-énedioïque, bis(1,1-diméthyléthyl) ester.**

En opérant de façon analogue à la préparation 18, au départ de 2,73 g ($6,47.10^{-3}$ mole) du composé obtenu selon la préparation 35 et de 2,5 g ($6,47.10^{-3}$ mole) d'acide [[(8-aminooctyl)imino]-méthylène]biscarbamique, bis(1,1-diméthyléthyl) ester, on obtient 2 g (Rendement = 39 %) du produit attendu sous forme d'huile jaune.

RMN $^1$H ($CDCl_3$) : 1,05 (d,3H) ; 1,1-1,8 (m,45H) ; 2,3-2,7 (m,4H) ; 3,15-3,30 (m,4H) ; 3,4 (td,2H) ; 3,45 (d,1H) ; 3,6 (d,1H) ; 3,6-3,75 (m,1H) ; 5,3-5,5 (sl,1H) ; 7,2-7,35 (m,5H) ; 7,35-7,6 (m,2H) ; 8,3 (t,1H) ; 11,5 (s,1H).

**PREPARATION 39**

**Acide 3-[[(1,1-diméthyléthoxy)carbonyl]amino]-24-méthyl-14,15-dioxo-2,4,13,16,21,25-hexaazahexacos-2-énedioïque, bis(1,1-diméthyléthyl) ester.**

En opérant de façon analogue à la préparation 15, au départ de 2 g ($2,53.10^{-3}$ mole) du composé obtenu selon la préparation 38, on obtient 1,75 g (Rendement = 99 %) du produit attendu sous forme d'huile incolore.

RMN $^1$H ($CDCl_3$) : 1,2 (dd,3H) ; 1,2-1,8 (m,45H) ; 2,45-2,9 (m,4H) ; 3,1-3,8 (m,8H) ; 4,7-4,85 (sl,1H) ; 7,4-7,5 (sl,1H) ; 7,7-7,85 (sl,1H) ; 8,3 (t,1H) ; 11,5 (s,1H).

**Exemple 11**

**N-[4-[[3-(amino)butyl]amino]butyl]-N'-[8[(aminoiminométhyl)amino]-octyl]-éthanediamide, tris(trifluoroacétate).**

En opérant de façon analogue au procédé de l'exemple 1, au départ de 1,75 g ($2,5.10^{-3}$ mole) du composé obtenu selon la préparation 39, on obtient 250 mg (Rendement = 13,5 %) du produit attendu sous forme d'un solide amorphe blanc.

RMN $^1$H (DMSO d6) : 1,2 (d,3H) ; 1,2-1,6 (m,16H) ; 1,6-1,8 (m,1H) ; 1,8-2,0 (m,1H) ; 2,8-3,2 (m,10H) ; 3,2-3,35 (m,1H) ; 6,7-7,4 (sl,3H) ; 7,55 (t,1H) ; 7,85-8,05 (sl,4H) ; 8,4-8,6 (sl,2H) ; 8,7 (t,1H) ; 8,77 (t,1H).
RMN $^{13}$C ($D_2O$): 15,00 ; 20,77 ; 23,12 ; 23,51 ; 23,74 ; 25,60 ; 25,91 ; 25,94 ; 25,98 ; 28,20 ; 36,53 ; 37,45 ; 38,97

; 41,60 ; 43,09 ; 45,11 ; 157,45 ; 161,55 ; 161,88.

**PREPARATION 40**

**Acide 3-[[(1,1-diméthyléthoxy)carbonyl]amino]-24-méthyl-21-phénylméthyl-12,15-dioxo-2,4,11,14,16,21,25-heptaazahexacos-2-énedioïque, bis(1,1-diméthyléthyl) ester.**

On dissout 2 g ($4,8.10^{-3}$ mole) d'acide 13-amino-3[[(1,1-diméthylethoxy)carbonyl)amino]-12-oxo-2,4,11-triazatridéc-2-énoïque, 1,1-diméthyléthyl ester dans 50 ml de dichlorométhane et on ajoute par fractions, à température ambiante, 1,6g ($5,3.10^{-3}$ mole) de bis(4-nitrophényl)carbonate. On maintient sous agitation pendant 5 heures à température ambiante, puis on ajoute 1,68 g ($4,8.10^{-3}$ mole) du composé obtenu selon la préparation 5, en solution dans 15 ml de dichlorométhane. On agite le milieu réaction ne à température ambiante pendant 16 heures, puis on concentre sous pression réduite. On purifie par chromatographie sur gel de silice et élution avec un mélange acétate d'éthyle/cyclohexane (4/6) v/v puis acétate d'éthyle/éthanol (9/1) v/v. On obtient ainsi 2,59 g (Rendement = 68 %) du produit attendu sous forme d'un solide jaune.

RMN $^1H$ ($CDCl_3$) : 1,0-1,1 (d,3H) ; 1,2-1,8 (m,41H) ; 2,3-2,7 (2m,4H) ; 3,1-3,4 (m,8H) ; 3,5-3,6 (m,1H) ; 3,9 (m,2H) ; 5,2 (m,1H) ; 5,7 (m,1H) ; 6,4 (m,1H) ; 6,8 (m,1H) ; 7,3 (m,5H) ; 8,3 (t,1H) ; 11,45 (s,1H).

**PREPARATION 41**

**Acide 3-[[(1,1-diméthyléthoxy)carbonyl]amino]-24-méthyl-12,15-dioxo-2,4,11,14,16,21,25-heptaazahexacos-2-énedioïque, bis(1,1-diméthyléthyl) ester, chlorhydrate.**

On soumet à l'hydrogénation sous pression atmosphérique, une solution de 2,52 g ($3,2.10^{-3}$ mole) du composé obtenu selon la préparation 40, dans 40 ml d'éthanol en présence de 200 mg de charbon palladié à 10 % et 0,1 ml d'acide chlorhydrique. Après 5 heures de réaction, le catalyseur est éliminé par filtration et la solution est concentrée sous pression réduite. Le résidu est repris avec de l'eau et du dichlorométhane et acidifié jusqu'à pH2. Après deux extractions à l'eau, les phases aqueuses rassemblées sont lyophilisées. Le solide amorphe (2.2 g) obtenu est traité sans autre purification dans la préparation suivante.

RMN $^1H$ ($CDCl_3$) : 1,0-1,1 (d,3H) ; 1,15-1,7 (m,39H) ; 1,8 (m,1H) ; 2,1 (m,1H) ; 2,8-3,1 (m,9H) ; 3,2-3,4 (2m,2H) ; 3,6 (s,2H) ; 7,3 (m,2H) ; 8,2 (t,2H) ; 7,8 (t,1H) ; 9 (m,1H) ; 11 (s,1H).

**Exemple 12**

**N-[4-[[3-(amino)butyl]amino]butyl]-N'[[[6-[(aminoiminométhyl)amino]-hexyl]amino]carbonyl]méthyl]-urée, tris(trifluoroacétate).**

En opérant de façon analogue au procédé de l'exemple 1, au départ de 2,2 g du composé obtenu selon la préparation 41, on obtient 1 g (Rendement = 43 %) du produit attendu sous forme d'un solide amorphe blanc.

RMN $^1H$ (DMSO d6) : 1,1-1,65 (m,15H) ; 1,7 (m,1H) ; 1,9 (m,1H) ; 3.0 (m,10H) ; 3,3 (m,1H) ; 3,6 (s,2H) ; 4,5-5,5 (sl,3H) ; 6,0-6,4 (m,2H) ; 6,9-7,5 (m,2H) ; 7,6 (t,1H) ; 7,8 (t,1H) ; 8 (s,2H) ; 8,6 (m,2H).
RMN $^{13}C$ ($D_2O$): 18,01 ; 23,69 ; 26,20 ; 26,31 ; 27,20 ; 28,56 ; 29,02 ; 31,22 ; 39,85 ; 39,95 ; 41,87 ; 43,98 ; 44,59 ; 46,10 ; 48,26 ; 157,90 ; 161,07 ; 173,72.

**PREPARATION 42**

**Acide (2-hydroxy-1(R)-méthyl-éthyl)carbamique, 1,1-diméthyléthyl ester.**

On prépare une solution de 24,3 g (0,323 mole) de 2(R)-amino-propanol dans 450 ml de tetrahydrofuranne et 8 ml d'eau. On ajoute 32,6 g (0,323 mole) de triéthylamine, puis lentement une solution de 70,5 g (0,323 mole) de dicarbonate de di-tert.-butyle (i.e. $O[CO_2C(CH_3)_3]$) dans 150 ml de tétrahydrofurane. On maintient le milieu réactionnel sous agitation pendant 1 heure à température ambiante puis on concentre sous pression réduite. On reprend le résidu huileux avec 400 ml d'éther éthylique et lave avec 2 fois 100 ml d'une solution d'acide chlorhydrique 0,1 N chargée en chlorure de sodium, puis par une solution de bicarbonate de sodium. La phase organique est séchée sur sulfate de magnésium et concentrée sous pression réduite. Le résidu est repris avec 200 ml de cyclohexane et cristallisé. Le produit brut est enfin recristallisé dans 350 ml de cyclohexane et on obtient 49,3 g (Rendement = 87 %) du produit attendu

sous forme de cristaux blancs.

**F = 60° C**
$[\alpha]^{22}_{D}$ = + 12,1° (c = 1,00 ; $CHCl_3$)

**PREPARATION 43**

**Acide [2-(méthylsulfonyloxy)-1(R)-méthyl-éthyl]carbamique, 1,1-diméthyléthyl ester.**

On dissout 55 g (0,314 mole) du produit obtenu selon la préparation 42 dans 600 ml de dichlorométhane et on ajoute 90 g (0.89 mole) de triéthylamine. On refroidit le mélange à -5° C et on ajoute lentement une solution de 51 g (0,445 mole) de chlorure de méthanesulfonyle dans 100 ml de dichlorométhane. On laisse ensuite revenir à température ambiante et on maintient sous agitation pendant 15 heures. Le milieu réactionnel est ensuite versé dans 200 ml d'eau glacée. La phase organique est lavée avec une solution de chlorure de sodium puis séchée sur sulfate de magnésium et concentrée sous pression réduite. On obtient ainsi 79 g (Rendement quantitatif) du produit attendu brut sous forme de cristaux orangés qui sont utilisés ainsi pour l'opération suivante. Le produit peut être purifé par recristallisation dans l'heptane.

**F = 76° C**
$[\alpha]^{23}_{D}$ = + 29,7° (c = 1,02 ; $CHCl_3$)

**PREPARATION 44**

**Acide (2-cyano-1(R)-méthyl-éthyl)carbamique, 1,1-diméthyléthyl ester.**

On dissout 79 g (0,31 mole) du composé obtenu selon la préparation 43 dans 600 ml de diméthylsulfoxyde et on ajoute 40,5 g (0,62 mole) de cyanure de potassium. On maintient ensuite le milieu réactionnel sous agitation à 50° C pendant 15 heures. On refroidit et on hydrolyse sur 600 ml d'eau glacée. On extrait par 4 fois 500 ml d'éther éthylique et on sèche les phases organiques sur sulfate de magnésium. Après concentration sous pression réduite, on purifie le produit brut par chromatographie sur gel de silice et élution avec un mélange méthylcyclohexane/acétate d'éthyle (8/2) v/v. On obtient ainsi 42 g d'huile qui cristallise. Après recristallisation dans un mélange de méthylcyclohexane et d'éther isopropylique, on obtient 33 g (Rendement = 57 %) du produit attendu sous forme de cristaux blancs.

**F = 70° C**
$[\alpha]^{23}_{D}$ = + 93,2° (c = 1,00 ; $CHCl_3$)

**PREPARATION 45**

**Acide (3-amino-1(R)-méthyl-propyl)carbamique, 1,1-diméthyléthyl ester.**

On prépare une solution de 23,64 g (0.128 mole) du composé obtenu selon la préparation 44 dans 500 ml d'éthanol, et on ajoute 10 ml d'une solution de NaOH 1N et 7 g de Nickel de Raney. Le mélange est agité sous une pression d'hydrogène de $20.10^5$ pascals, pendant 48 heures. Après filtration du catalyseur, le filtrat est neutralisé par addition d'acide chlorhydrique 1N et concentré sous pression réduite. Le résidu est purifié par chromatographie sur gel de silice et élution avec un mélange méthylcyclohexane/acétate d'éthyle/ammoniaque (7,5/2/0,5) v/v. On obtient ainsi 22,1 g (Rendement = 91,5 %) du produit pur attendu qui cristallise.

**F = 73° C**
$[\alpha]^{23,5}_{D}$ = + 12,0° (c = 1,00 ; $CHCl_3$)

**PREPARATION 46**

**Acide [3-(phénylméthylamino)-1(R)-méthyl-propyl]carbamique, 1,1-diméthyléthyl ester.**

On prépare une solution de 22,1 g (0,117 mole) du composé obtenu selon la préparation 45 dans 300 ml d'éther éthylique et on ajoute 20 g de tamis moléculaire 3 Å puis 12,47 g (0.117 mole) de benzaldéhyde. On maintient le mélange sous agitation pendant 15 heures à température ambiante puis on élimine le tamis moléculaire par filtration et on concentre sous pression réduite. On obtient ainsi 32,6 g de l'imine intermédiaire que l'on dissout dans 350 ml d'éthanol. On ajoute à la solution 6,7 g (0,177 mole) de borohydrure de sodium par fractions, en maintenant la température à

environ 10°C. On maintient ensuite 3 heures sous agitation puis on concentre sous pression réduite. Le résidu est repris dans 500 ml d'éther éthylique et la phase organique est lavée à l'eau, séchée sur sulfate de magnésium et concentrée sous pression réduite. Après recristallisation du résidu dans du cyclohexane on obtient 32,5 g (Rendement = 98 %) du produit attendu sous forme de cristaux jaunes.

**F = 79° C**
$[\alpha]^{23}_D$ = - 5,2° (c = 2,00 ; $CHCl_3$)

**PREPARATION 47**

**Acide 9-cyano-3(R)-méthyl-6-phénylméthyl-2,6-diazanonanoïque, 1,1-diméthyléthyl ester.**

On prépare une solution de 32 g (0.115 mole) du composé obtenu selon la préparation 46 et 18,2 g (0,175 mole) de 4-chlorobutyronitrile dans 300 ml de butanol. On ajoute 14,6 g (0.138 mole) de carbonate de sodium et 4,8 g d'iodure de potassium et on porte à reflux sous agitation pendant 15 heures. On concentre sous pression réduite et purifie le résidu par chromatographie sur gel de silice et élution avec un mélange méthylcyclohexane/acétate d'éthyle (8/2 puis 1/1) v/v. On obtient ainsi 39 g (Rendement = 98 %) du produit attendu sous forme d'une huile jaune très visqueuse.

$[\alpha]^{23}_D$ = - 6,9° (c = 2,00 ; $CHCl_3$)

**PREPARATION 48**

**Acide 10-amino-3(R)-méthyl-6-phénylméthyl-2,6-diazadécanoïque, 1,1-diméthyléthyl ester.**

En opérant de façon analogue à la préparation 45, en travaillant sous une pression d'hydrogène de $3,5.10^5$ pascals, au départ de 32,8 g ($95.10^{-3}$ mole) du composé obtenu selon la préparation 45, on obtient 33 g (Rendement = 99 %) du produit attendu sous forme d'une huile visqueuse incolore.

$[\alpha]^{23}_D$ = - 1,9° (c = 1,00 ; $CHCl_3$)
RMN $^1H$ ($CDCl_3$) : 1,04 (d,3H) ; 1,35-1,8 (m,17H) ; 2,3-2,5 (m,4H) ; 2,64 (t,2H) ; 3,44 (d,1H) ; 3,61 (d,1H) ; 3,62-3,8 (m,1H) ; 5,6-5,8 (sl,1H) ; 7,2-7,35 (m,5H).

**PREPARATION 49**

**Acide 10-amino-3(S)-méthyl-6-phénylméthyl-2,6-diazadécanoïque, 1,1-diméthyléthyl ester.**

Par une suite de réactions analogues aux préparations 42 à 48, au départ de 2(S)-amino-propanol, on obtient le dérivé chiral de configuration S attendu.

$[\alpha]^{23}_D$ = + 1,6° (c = 1,20 ; $CHCl_3$)

**PREPARATION 50**

**Acide 3(R)-méthyl-10-[2,4-dioxo-oxazolidine-3-yl]-6-phénylméthyl-2,6-diazadécanoïque, 1,1-diméthyléthyl ester.**

En opérant de façon analogue à la préparation 10, au départ de 1,8 g ($5,15.10^{-3}$ mole) du composé obtenu selon la préparation 48, on obtient le produit attendu sous forme de cristaux blancs avec un rendement de 90 %.

**F = 62° C**
$[\alpha]^{22}_D$ = - 1° (c = 1,00 ; $CHCl_3$)

**PREPARATION 51**

**Acide 3(S)-méthyl-10-[2,4-dioxo-oxazolidine-3-yl]-6-phénylméthyl-2,6-diazadécanoïque, 1,1-diméthyléthyl ester.**

Selon un procédé identique à la préparation 50, au départ du composé de la préparation 49, on obtient le produit

attendu sous forme d'huile incolore.

$[\alpha]^{22}_D$ = + 0,5° (c = 1,00 ; $CHCl_3$)
RMN $^1H$ ($CDCl_3$) : 1,05 (d,3H) ; 1,35-1,65 (m,15H) ; 2,35-2,65 (m,4H) ; 3,4-3,75 (m,5H) ; 4,67 (s,2H) ; 5,2-5,4 (sl,1H) ; 7,2-7,4 (m,5H).

**PREPARATION 52**

**Acide 3(R)-méthyl-6-phénylméthyl-13-oxa-12-oxo-2,6,11-triazapentanedioïque, 1-(1,1-diméthyléthyl) ester.**

En opérant de façon analogue à la préparation 11, au départ de 2 g ($4,6.10^{-3}$ mole) du composé obtenu selon la préparation 50, on obtient 2,1 g (Rendement = 99 %) du produit attendu sous forme de cristaux blancs amorphes.

$[\alpha]^{22}_D$ = - 3,9° (c = 1.00 ; $CHCl_3$)
RMN $^1H$ ($CDCl_3$) : 1,17 (d,3H) ; 1,25-2,1 (m,15H) ; 2,35-3,3 (m,8H) ; 3,45-3,7 (m,1H) ; 4-4,35 (m,2H) ; 4,8-5,2 (m,1H) ; 7,15-7,8 (m,6H) ; 12,1-12,7 (sl,1H).

**PREPARATION 53**

**Acide 3(S)-méthyl-6-phénylméthyl-13-oxa-12-oxo-2,6,11-triazapentanedioïque, 1-(1,1-diméthyléthyl) ester.**

En opérant de façon analogue à la préparation 52, au départ du composé obtenu selon la préparation 51, on obtient le produit attendu sous forme d'un solide blanc amorphe.

$[\alpha]^{22}_D$ = + 6,0° (c = 0,41 ; $CHCl_3$)

**PREPARATION 54**

**Acide 3-[[(1,1-diméthyléthoxy)carbonyl]amino]-24(R)-méthyl-21-phénylmethyl-14-oxa-12,15-dioxo-2,4,11,16,21,25-hexaazahexacos-2-ènedioïque, bis(1,1-diméthyléthyl) ester.**

En opérant de façon analogue au procédé de la préparation 16, au départ de 2,08 g ($4,61.10^{-3}$ mole) du composé obtenu à la préparation 52, on obtient 3.6 g (Rendement = 99 %) du produit attendu sous forme d'huile incolore.

$[\alpha]^{22}_D$ = - 2,3° (c = 1,00 ; $CHCl_3$)
RMN $^1H$ ($CDCl_3$) : 1,01 (d,3H) ; 1,3-1,8 (m,41H) ; 2,3-2,5 (m,3H) ; 2,5-2,65 (m,1H) ; 3,05-3,2 (m,2H) ; 3,2-3,3 (m,2H) ; 3,35-3,5 (m,3H) ; 3,6 (d,1H) ; 3,65-3,8 (m,1H) ; 4,5 (s,2H) ; 5,35-5,6 (m,2H) ; 6,25-6,4 (sl,1H) ; 7,2-7,35 (m,5H) ; 8,3 (t,1H) ; 11,5 (s,1H).

**PREPARATION 55**

**Acide 3-[[(1,1-diméthyléthoxy)carbonyl]amino]-24(S)-méthyl-21-phénylméthyl-14-oxa-12,15-dioxo-2,4,11,16,21,25-hexaazahexacos-2-ènedioïque, bis(1,1-diméthyléthyl) ester.**

En opérant de façon analogue à la préparation 54, au départ de 1,56 g du composé obtenu selon la préparation 53, on obtient le produit attendu 2,02 g (Rendement = 74 %) sous forme d'une huile incolore.

$[\alpha]^{22}_D$ = + 1,8° (c = 1,00 ; $CHCl_3$)

**PREPARATION 56**

**Acide 3-[[(1,1-diméthyléthoxy)carbonyl]amino]-24(R)-méthyl-14-oxa-12,15-dioxo-2,4,11,16,21,25-hexaazahexacos-2-ènedioïque, bis(1,1-diméthyléthyl) ester.**

En opérant de façon analogue à la préparation 15, au départ de 3,6 g ($4,5.10^{-3}$ mole) du composé obtenu selon la préparation 54, on obtient 3,2 g du produit attendu (Rendement = 99 %) sous forme d'une huile jaune clair.

$[\alpha]^{22}_D$ = - 4,4° (c = 1,00 ; $CHCl_3$)
RMN $^1H$ ($CDCl_3$) : 1,18 (d,3H) ; 1,25-1,95 (m,42H) ; 2,6-2,8 (m,4H) ; 3,1-3,45 (m,6H) ; 3,7-3,9 (m,1H) ; 4,54 (s,2H)

; 4,65-4,8 (sl,1H) ; 5,8-6,1 (sl,1H) ; 6,4-6,6 (sl,1H) ; 8,3 (t,1H) ; 11,5 (s,1H).

**PREPARATION 57**

**Acide 3-[[(1,1-diméthyléthoxy)carbonyl]amino]-24(S)-méthyl-14-oxa-12,15-dioxo-2,4,11,16,21,25-hexaazahexacos-2-ènedioïque, bis(1,1-diméthyléthyl) ester.**

En opérant de façon analogue à la préparation 56, au départ de 2.0 g ($2,5.10^{-3}$ mole) du composé obtenu à la préparation 55, on obtient le produit attendu sous forme d'une huile jaune.

$[\alpha]^{22}_D$ = + 5,8° (c = 1,00 ; $CHCl_3$)

**Exemple 13**

**2-[[[4-[[3(R)-(amino)butyl]amino]butyl]amino]carbonyloxy]-N-[6-[(aminoiminométhyl)amino]hexyl]acétamide, tris(trifluoroacétate).**

En opérant de façon analogue au procédé de l'exemple 1, au départ de 3,2 g ($4,56.10^{-3}$ mole) du composé obtenu selon la préparation 56, on obtient 2,48 g (Rendement = 73 %) du produit attendu sous forme de solide blanc amorphe.

$[\alpha]^{22}_D$ = + 1,1° (c = 2.00 ; $CH_3OH$)
RMN $^1H$ (DMSO d6) : 1,18 (d,3H) ; 1,25-1,65 (m,12H) ; 1,65-1,85 (m,1H) ; 1,85-2,0 (m,1H) ; 2,85-3,15 (m,10H) ; 3,2-3,35 (m,1H) ; 4,33 (s,2H) ; 6,8-7,3 (sl,3H) ; 7,32 (t,1H) ; 7,62 (t,1H) ; 7,86 (t,1H) ; 7,9-8,05 (sl,4H) ; 8,5-8,7 (sl,2H).
RMN$^{13}C$ ($D_2O$) : 18,01 ; 23,64 ; 26,19 ; 26,28 ; 26,76 ; 28,56 ; 28,93 ; 31,21 ; 39,77 ; 40,58 ; 41,87 ; 44,60 ; 46,10 ; 48,21 ; 63,75 ; 157,55 ; 157,89 ; 171,44.

**Exemple 14**

**2-[[[4-[[3(S)-(amino)butyl]amino]butyl]amino]carbonyloxy]-N-[6-[(aminoiminométhyl)amino]hexyl]acétamide, tris(trifluoroacétate).**

En opérant de façon analogue à l'exemple 13, au départ de 1,75 g ($2,5.10^{-3}$ mole) du composé obtenu selon la préparation 57, on obtient 1,5 g (Rendement = 82 %) du produit attendu sous forme d'un solide amorphe.

$[\alpha]^{22}_D$ = - 0,95° (c = 2,00 ; $CH_3OH$)

**PREPARATION 58**

**Acide 3-[[(1,1-diméthyléthoxy)carbonyl]amino]-23(R)-méthyl-12,14-dioxo-20-phénylmethyl-2,4,11,15,20,24-hexaazapentacos-2-ènedioïque, bis(1,1-diméthyléthyl) ester.**

En opérant de façon analogue à la préparation 18, au départ de 4.88 g ($11.10^{-3}$ mole) du composé obtenu selon la préparation 7 et de 2,75 g ($7,88.10^{-3}$ mole) du composé obtenu à la préparation 48, on obtient 4,9 g (Rendement = 80 %) du produit attendu sous forme d'une huile visqueuse.

$[\alpha]^{23}_D$ = -4,1° (c = 1,00 ; $CHCl_3$)
RMN $^1H$ ($CDCl_3$) : 1,02 (d,3H) ; 1,25-1,85 (m,41H) ; 2,25-2,7 (m,4H) ; 3,05-3,8 (m,11H) ; 5,2-5,4 (sl,1H) ; 7-7,15 (sl,1H) ; 7,15-7,4 (m,6H) ; 8,3 (t,1H) ; 11,5 (s,1H).

**PREPARATION 59**

**Acide 3-[[(1,1-diméthyléthoxy)carbonyl]amino]-23(R)-méthyl-12,14-dioxo-2,4,11,15,20,24-hexaazapentacos-2-ènedioïque, bis(1,1-diméthyléthyl) ester.**

En opérant de façon analogue à la préparation 15, au départ de 4,9 g ($6,32.10^{-3}$ mole) du composé obtenu selon la préparation 58, on obtient 4,32 g (Rendement = 99 %) du produit attendu sous forme huileuse.

$[\alpha]^{23}_D$ = - 2,8° (c = 1,00 ; $CHCl_3$)

RMN $^1H$ ($CDCl_3$) : 1,2 (d,3H) ; 1,25-2,0 (m,42H) ; 2,6-2,8 (m,4H) ; 3,16 (s,2H) ; 3,2-3,55 (m,6H) ; 3,6-3,85 (m,1H) ; 4,75-4,95 (sl,1H) ; 7,1-7,35 (sl,1H) ; 7,65-7,8 (sl,1H) ; 8,3 (t, 1H) ; 11,5 (s, 1H).

**Exemple 15**

**N-[4-[[3(R)-(amino)butyl]amino]butyl]-N'-[6-[(aminoiminométhyl)-amino]hexyl]-propanediamide, tris(trifluoroacétate).**

En opérant de façon analogue au procédé de l'exemple 1, au départ de 4,3 g (6,27.10-3 mole) du composé obtenu selon la préparation 59, on obtient après purification par chromatographie sur gel de silice RP18 et élution avec un mélange eau/acétonitrile/acide trifluoroacétique (8/1/1) v/v, 2,1 g (rendement = 46 %) du produit attendu sous forme d'un solide amorphe transparent.

$[\alpha]^{23}_D$ = + 1,1° (c = 1,00 ; $CH_3OH$)
RMN $^1H$ (DMSO d6) : 1,2 (d,3H) ; 1,2-1,65 (m,12H) ; 1,65-1,85 (m,1H) ; 1,85-2 (m,1H) ; 2,8-3,15 (m,12H) ; 3,2-3,35 (m,1H) ; 6,7-7,5 (sl,3H) ; 7,62 (t,1H) ; 7,7-8,2 (m,6H) ; 8,3-8,8 (sl,2H) .
RMN$^{13}C$ ($D_2O$) : 18,02 ; 23,71 ; 26,23 ; 26,35 ; 28,56 ; 28,74 ; 28,84 ; 31,23 ; 39,50 ; 40,31 ; 41,88 ; 44,30 ; 44,61 ; 46,10 ; 48,15 ; 115,25 ; 119,11 ; 158,30 ; 170,02 ; 170,34.

L'activité immunosuppressive des produits selon l'invention a été mise en évidence à l'aide d'un test de réaction du greffon contre l'hôte. Des souris mâles B6D2F1 (hybrides de première génération C57B1/6 x DBA/2 ) sont immunodéprimées par une injection intrapéritonéale (i.p.) de cyclophosphamide. Trois jours après (jour 0 de l'expérience : J0), elles reçoivent, par voie intraveineuse, 4 x $10^7$ splénocytes de souris C57B1/6. Les animaux sont ensuite répartis par lot de 8 au minimum et reçoivent un traitement journalier de J1 à J5 et de J7 à J10 par voie i.p. Le groupe contrôle reçoit le véhicule seul. La mortalité est suivie jusqu'à J60. Les résultats, exprimés par la valeur moyenne de la survie en jours à la dose indiquée, sont regroupés dans le tableau I où les valeurs données sont significatives selon le test de Logrank (probabilité inférieure ou égale à 5 %). A fin de comparaison, on a également indiqué dans le tableau I les valeurs obtenues avec les produits connus de l'art antérieur : la 15-déoxyspergualine (15-DSG), la cyclosporine A qui est actuellement l'immunosuppresseur de référence utilisé en thérapeutique, et le produit de l'exemple 1 décrit dans EP-A-0 105 193. Il ressort de cette comparaison que les produits selon l'invention sont jusqu'à 100 fois plus actifs que les produits connus de l'art antérieur. Notamment, les produits selon l'invention présentent une activité significative dès 0,3 mg/kg (plus faible dose testée) alors que le produit de comparaison de l'exemple 1 de EP-A-0 105 193 ne présente une activité significative qu'à partir de 1 mg/kg, et la cyclosporine A qu'à partir de 25 mg/kg.

De plus, les composés selon l'invention présentent une stabilité en solution nettement plus grande que les produits connus de l'art antérieur, notamment la 15-déoxyspergualine.

Les produits selon l'invention sont utiles en thérapeutique en tant qu'agents immunosuppresseurs curatifs ou préventifs, notamment dans la prévention du rejet d'organes allogéniques ou xénogéniques vascularisés ou non, de la réaction du greffon contre l'hôte suite à une greffe vascularisée ou non, dans le traitement des maladies auto-immunes génétiquement définies ou acquises (comme par exemple le lupus érythémateux disséminé, la sclérose en plaques, la polyarthrite rhumatoïde), des maladies inflammatoires chroniques telles que par exemple les rhumatismes articulaires ainsi que dans toutes les pathologies où un désordre immunitaire apparaît être la cause ou le facteur responsable du maintien d'un état clinique dégradé.

Les produits selon l'invention peuvent également être administrés en complément de drogues anticancéreuses cytotoxiques afin d'en limiter les effets secondaires et en complément de l'administration de produits issus des biotechnologies, notamment les cytokines recombinantes, les anticorps mono- et polyclonaux afin de diminuer l'apparition des anticorps protecteurs produits par le patient.

Les produits selon l'invention peuvent être utilisés dans le traitement curatif de parasitoses, en particulier dans le cas du paludisme.

Les produits selon l'invention peuvent être administrés par voie orale, par voie injectable (notamment intra-musculaire ou intraveineuse), par voie topique (notamment sous forme de crème pour application locale, de gouttes occulaires), par voie transdermique, par voie rectable sous forme de suppositoire ou par inhalation.

Les produits selon l'invention trouvent également leur utilité en tant que réactifs pharmacologiques, notamment dans l'étude des maladies auto-immunes.

Dans le tableau I, tous les produits cités des exemples selon l'invention sont sous forme de tris(trifluoroacétate).

TABLEAU I

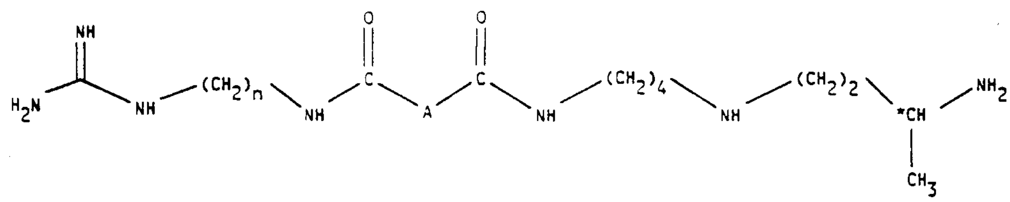

(I)

| Exemple | A | n | Chiralité | Activités<br>Dose (mg/kg) | Survie (jours) |
|---|---|---|---|---|---|
| 1 | $-CH_2-$ | 6 | racémique | 3 | 60 |
| 2 | $-CH_2-O-$ | 6 | racémique | 0,3 | 53 |
| 3 | $-CH_2-$ | 8 | racémique | 3 | 57 |
| 4 | -CH(OH)- | 6 | mélange de diastéréoisomères | 3 | 60 |
| 5 | -CH(OH)- | 8 | mélange de diastéréoisomères | 3 | 60 |
| 6 | $-CH(OCH_3)-$ | 6 | mélange de diastéréoisomères | 3 | 58 |
| 7 | $-CH(OCH_3)-$ | 8 | mélange de diastéréoisomères | - | - |
| 8 | -CHF- | 6 | mélange de diastéréoisomères | - | - |
| 9 | -CHF- | 8 | mélange de diastéréoisomères | 3 | 60 |
| 10 | simple liaison | 6 | racémique | 0,3 | 38 |
| 11 | simple liaison | 8 | racémique | 3 | 60 |

Tableau I (fin)

| Exemple | A | n | Chiralité | Activités Dose (mg/kg) | Survie (jours) |
|---|---|---|---|---|---|
| 12 | $-CH_2-NH-$ | 6 | racémique | 1 | 56 |
| 13 | $-CH_2-O$ | 6 | chiral (forme R) | 0,3 | 60 |
| 15 | $-CH_2-$ | 6 | chiral (forme R) | - | - |
| 15-DSG | | | | 1 | 43 |
| cyclosporine A | | | | 25 | 36 |
| Ex 1 de EP-A-0105193 | | | | 1 | 32 |

## Revendications

1. Composé appartenant à la famille des analogues de 15-déoxyspergualine, caractérisé en ce qu'il est choisi parmi l'ensemble constitué par :

   **(i)** les composés de formule :

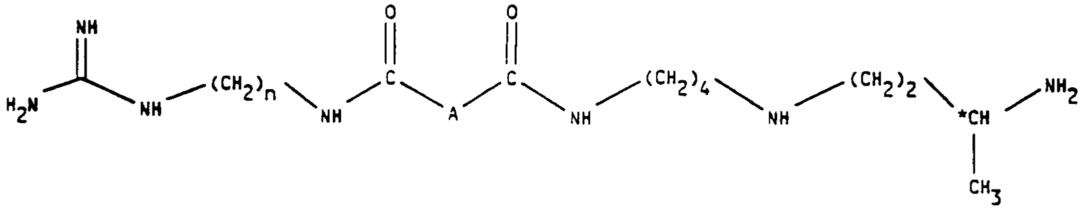

(I)

dans laquelle :

A représente une liaison simple, un groupe $-CH_2-$, un groupe $-CH_2O-$, un groupe-$CH_2NH-$, un groupe -CH(OH)-, un groupe - CHF- ou un groupe $-CH(OCH_3)-$,
n est égal à 6 ou 8 ; et,

**(ii)** leurs sels d'addition.

2. Composé selon la revendication 1, dans lequel A représente un groupe $CH_2$ ou un groupe $CH_2O$, et ses sels d'addition.

3. Composé selon la revendication 1, dans lequel *C représente un carbone de configuration (R) ou (R,S), et ses sels d'addition.

4. 2-[[[4-[[3-(amino)butyl]amino]butyl]amino]carbonyloxy]-N-[6-[(amino-iminométhyl)amino]hexyl]acétamide, tris(trifluoroacétate).

5. 2-[[[4-[[3(R)-(amino)butyl]amino]butyl]amino]carbonyloxy]-N-[6-[(aminoiminométhyl)amino]hexyl]acétamide, tris(trifluoroacétate).

6. Procédé de préparation d'un composé de formule I ou de l'un de ses sels d'addition selon la revendication 1, ledit procédé étant caractérisé en ce qu'il comprend la déprotection d'un composé de formule :

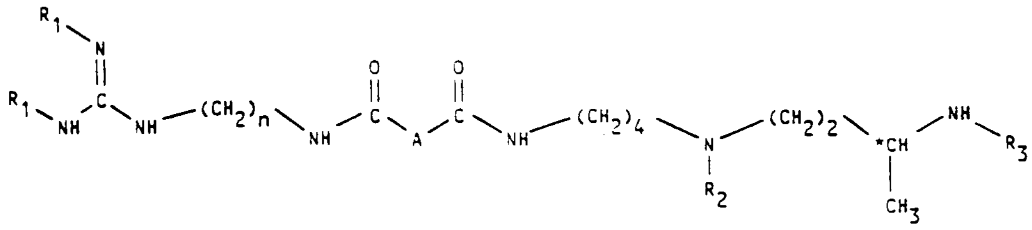

(II)

dans laquelle :

A représente une simple liaison, un groupe $CH_2$, un groupe CHF. un groupe $CH(OCH_3)$, un groupe CH(OH), un groupe $CH(OCH_2C_6H_5)$, un groupe $CH_2O$ ou un groupe $CH_2NH$,
n est égal à 6 ou 8, et
$R_1$, $R_2$, $R_3$, identiques ou différents, représentent chacun un groupe amino-protecteur de type alkyloxycarbonyle, benzyloxycarbonyle ou benzyle.

ladite déprotection comportant notamment

(α) le traitement dudit composé de formule II avec un acide fort quand l'un au moins des $R_1$, $R_2$ ou $R_3$ représente un groupe du type oxycarbonyle, et/ou
(β) l'hydrogénation catalytique dudit composé de formule II quand l'un au moins des $R_1$, $R_2$ ou $R_3$ représente un groupe du type benzyle ou quand A représente le groupe $CH(OCH_2C_6H_5)$,

pour obtenir le composé de formule I correspondant sous forme de base libre ou de l'un de ses sels d'addition.

7. Procédé selon la revendication 6, ledit procédé qui met en oeuvre l'obtention dudit composé de formule II, étant caractérisé en ce qu'il est choisi parmi l'ensemble constitué par :

**(a)** la variante A qui comprend les étapes consistant à :

**(i)** condenser un composé de formule :

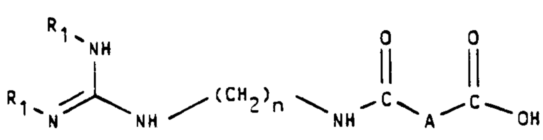

(III)

dans laquelle :

n est égal à 6 ou 8,
A représente le groupe $CH_2$, $CH(OCH_3)$, $CH(OCH_2\text{-}C_6H_5)$, CHF, ou une simple liaison,

$R_1$ représente un groupe amino-protecteur,

sur une amine de formule :

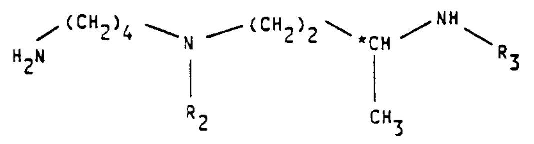

**(IV)**

dans laquelle :

$R_2$ et $R_3$, identiques ou différents, représentent chacun un groupe amino-protecteur,

par activation de l'acide :

- soit à l'aide d'un agent de couplage de type carbodiimide en présence d'un agent nucléophile dans un solvant organique, et à une temperature comprise entre 0°C et 40° C,
- soit par formation d'un anhydride mixte, notamment au moyen de chloroformiate d'isobutyle en présence d'un agent basique, dans un solvant organique, et à une température comprise entre -35°C et + 20°C,

à raison de 1 mole du composé III pour environ 1 mole du composé IV, pour obtenir un composé de formule:

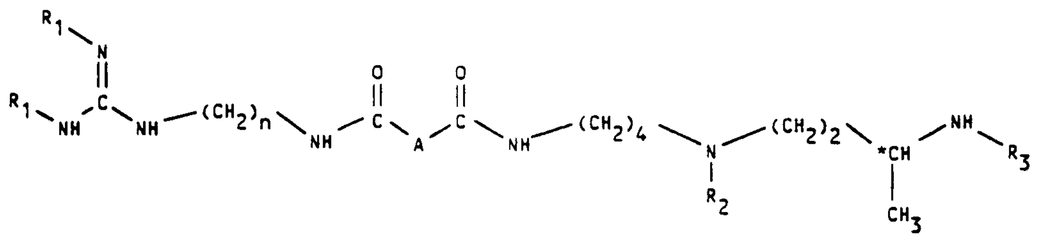

**(II)**

dans laquelle n, A, $R_1$, $R_2$ et $R_3$ ont les significations indiquées ci-dessus ;
**(ii)** déprotéger le composé de formule II obtenu à l'étape (i) au moyen de un ou de plusieurs traitements à l'aide d'un acide fort et/ou d'une hydrogénation catalytique, pour le remplacement de $R_1$, $R_2$ et $R_3$ par un atome d'hydrogène et obtenir un composé de formule I dans laquelle :

A représente une simple liaison , $CH_2$, CHF, $CHOCH_3$ ou CH(OH),
n est égal à 6 ou 8 ; et,

**(iii)** si nécessaire obtenir le composé de formule I sous forme de base libre par action d'une base forte, puis obtenir à partir de ladite base libre les autres sels d'addition ;

**(b)** la variante B qui comprend les étapes consistant à :

**(i)** faire réagir un composé de formule :

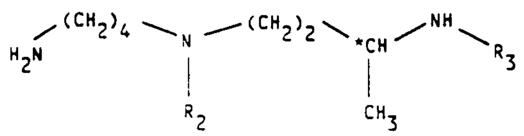

(IV)

dans laquelle :

$R_2$ et $R_3$, identiques ou différents, représentent chacun un groupe amino-protecteur de type oxycarbonyle ou benzyle,

avec un acide ou un chlorure d'acide de formule :

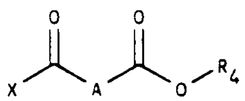

(V)

dans laquelle :

X représente un atome de chlore ou un groupe OH,
A représente une simple liaison, un groupe $CH_2$, un groupe CHF, un groupe $CH(OCH_3)$ ou un groupe $CH(OCH_2C_6H_5)$,
$R_4$ représente un groupe alkyle en $C_1$-$C_3$, linéaire ou ramifié, ou un groupe phénylméthyle,

dans un solvant organique, en présence d'un activateur de groupe carboxylique et d'un agent nucléophile, lorsque X représente le groupe OH, ou en présence d'une amine tertiaire, lorsque X représente un atome de chlore, à une température comprise entre 0°C et 40° C, à raison de 1 mole de IV pour environ 1 mole de V, pour obtenir un composé de formule :

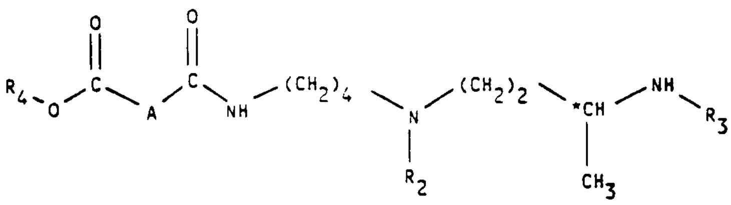

(VI)

dans laquelle A, $R_2$, $R_3$ et $R_4$ ont les significations indiquées ci-dessus ;
**(ii)** saponifier le composé de formule VI ainsi obtenu, dans un solvant organique, en présence d'une base forte pour obtenir un composé de formule :

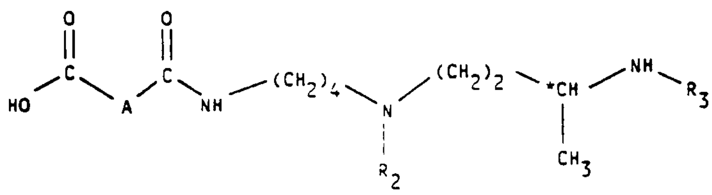

(VII)

dans laquelle A, $R_2$ et $R_3$ ont les significations indiquées ci-dessus ;

**(iii)** condenser le composé de formule VII ainsi obtenu, sur une amine de formule :

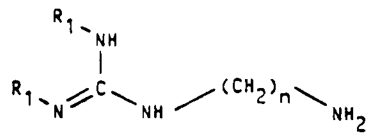

**(VIII)**

dans laquelle :

n est égal à 6 ou 8, et

$R_1$ représente un groupe amino-protecteur, dans des conditions identiques à celles de la variante A ci-dessus. pour obtenir un composé de formule:

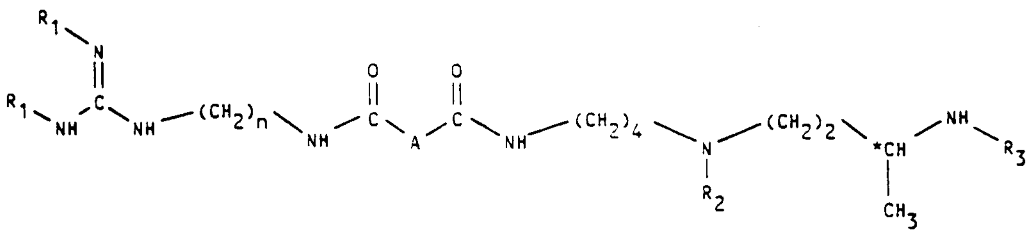

**(II)**

dans laquelle A, n, $R_1$, $R_2$ et $R_3$ ont les significations indiquées ci-dessus ;

**(iv)** déprotéger le composé de formule II obtenu à l'étape (iii) au moyen de un ou de plusieurs traitements à l'aide d'un acide fort et/ou d'une hydrogénation catalytique, pour le remplacement de $R_1$, $R_2$ et $R_3$ par un atome d'hydrogène et obtenir un composé de formule I dans laquelle :

A représente une simple liaison , $CH_2$, CHF, $CHOCH_3$ ou CH(OH), et

n est égal à 6 ou 8 ; et,

**(v)** si nécessaire, obtenir le composé de formule I sous forme de base libre par action d'une base forte, puis obtenir à partir de ladite base libre les autres sels d'addition ;

**(c)** la variante C qui comprend les étapes consistant à :

**(i)** acyler l'extrémité $NH_2$ terminale d'une base de formule :

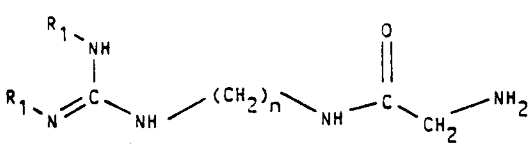

**(IX)**

dans laquelle $R_1$ représente un groupe amino-protecteur, et n est égal à 6 ou 8,

par un chloroformiate ou un carbonate symétrique dans un solvant inerte, à température ambiante (15-25°C).

**(ii)** aminolyser le composé ainsi obtenu par une amine de formule :

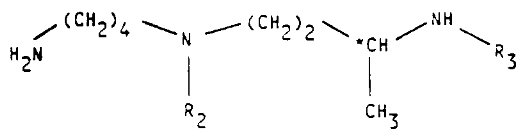

(IV)

dans laquelle $R_2$ et $R_3$, identiques ou différents, représentent chacun un groupe amino-protecteur, dans un solvant inerte, pour obtenir un composé de formule :

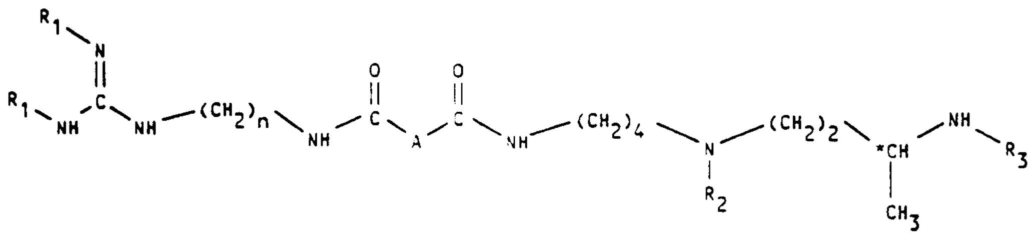

(II)

dans laquelle n, $R_1$, $R_2$ et $R_3$ ont les mêmes significations que ci-dessus et A représente le groupe -$CH_2NH$-;

**(iii)** déprotéger le composé de formule II obtenu à l'étape (ii) au moyen de un ou de plusieurs traitements à l'aide d'un acide fort et/ou d'une hydrogénation catalytique, pour le remplacement de $R_1$, $R_2$ et $R_3$ par un atome d'hydrogène et obtenir un composé de formule I dans laquelle :

A représente le groupe $CH_2NH$, et
n est égal à 6 ou 8 ; et,

**(iv)** si nécessaire, obtenir le composé de formule I sous forme de base libre par action d'une base forte, puis obtenir à partir de ladite base libre les autres sels d'addition ; et,

**(d)** la variante D qui comprend les étapes consistant à :

**(i)** faire réagir une amine de formule :

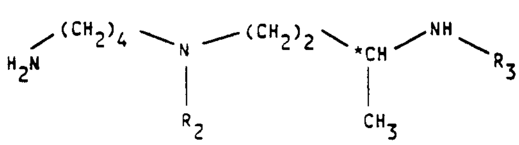

(IV)

dans laquelle $R_2$ et $R_3$, identiques ou différents, représentent chacun un groupe amino-protecteur, avec un carbonate de formule :

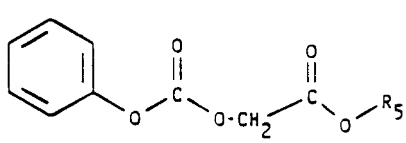

(X)

dans laquelle $R_5$ représente un groupe alkyle en $C_1$-$C_3$ ou un groupe phénylméthyle, dans un solvant

inerte, à une température comprise entre la température ambiante et la température de reflux du milieu réactionnel, à raison de 1 mole de IV pour environ 1 mole de X, pour obtenir un composé de formule :

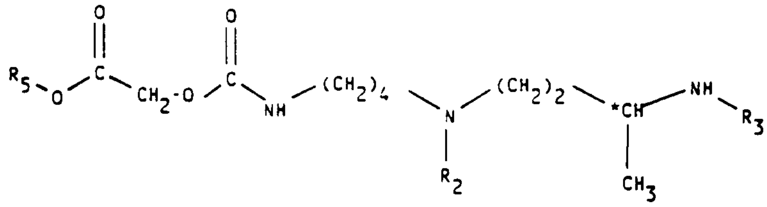

(XI)

dans laquelle $R_2$, $R_3$ et $R_5$ ont la même signification que ci-dessus. ou un composé de formule :

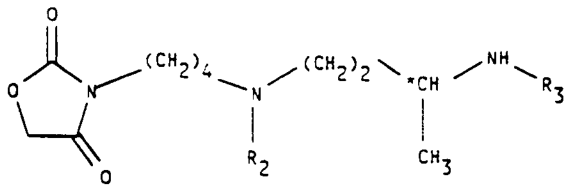

(XI')

dans laquelle $R_2$ et $R_3$ ont la même signification que ci-dessus ;

**(ii)** saponifier le composé XI ou XI' ainsi obtenu dans un solvant organique en présence d'une base forte, pour obtenir un composé de formule :

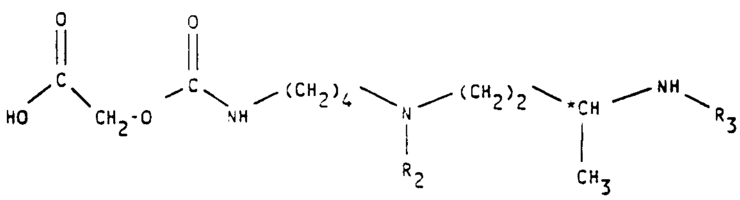

(XII)

dans laquelle $R_2$ et $R_3$ ont la même signification que ci-dessus ;

**(iii)** condenser le composé de formule XII ainsi obtenu, sur une amine de formule :

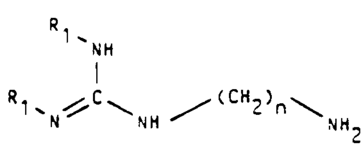

(VIII)

dans laquelle n est égal à 6 ou 8 et $R_1$ représente un groupe aminoprotecteur, dans des conditions identiques à celles décrites pour la variante A ci-dessus, pour obtenir un composé de formule :

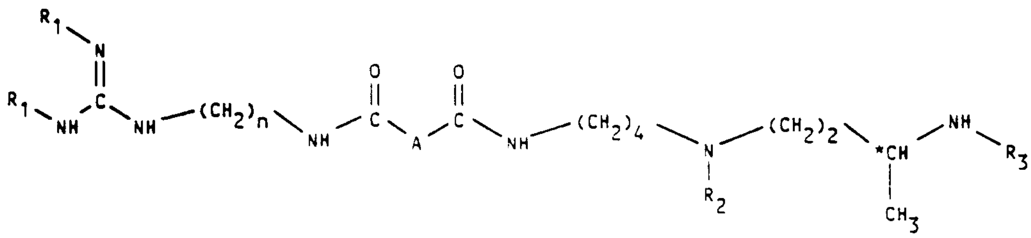

(II)

dans laquelle n, $R_1$, $R_2$ et $R_3$ ont les mêmes significations que ci-dessus et A représente un groupe $CH_2O$ ;

**(iv)** déprotéger le composé de formule II obtenu à l'étape (iii) au moyen de un ou de plusieurs traitements à l'aide d'un acide fort et/ou d'une hydrogénation catalytique, pour le remplacement de $R_1$, $R_2$ et $R_3$ par un atome d'hydrogène et obtenir un composé de formule I dans laquelle :

A représente un groupe $CH_2O$, et
n est égal à 6 ou 8 ; et,

**(v)** si nécessaire, obtenir le composé de formule I sous forme de base libre par action d'une base forte, puis obtenir à partir de ladite base libre les autres sels d'addition.

**8.** Composition thérapeutique caractérisée en ce qu'elle renferme, en association avec un excipient physiologiquement acceptable, au moins un composé choisi parmi l'ensemble constitué par les composés de formule I et leurs sels d'addition non toxiques selon la revendication 1.

**9.** Composé intermédiaire, utile pour la synthèse d'un composé de formule I selon la revendication 1, caractérisé en ce qu'il est choisi parmi l'ensemble constitué par les composés de formule :

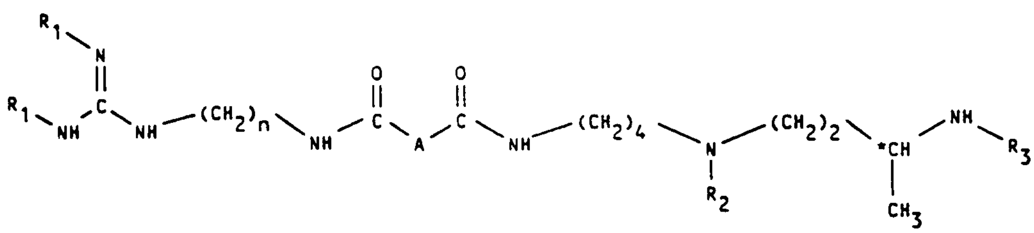

(II)

dans laquelle :

A représente une simple liaison, un groupe $CH_2$, un groupe CHF, un groupe $CH(OCH_3)$, un groupe CH(OH), un groupe $CH(OCH_2C_6H_5)$, un groupe $CH_2O$ ou un groupe $CH_2NH$,
n est égal à 6 ou 8, et
$R_1$, $R_2$, $R_3$, identiques ou différents, représentent chacun un groupe amino-protecteur du type alkyloxycarbonyle, benzyloxycarbonyle ou benzyle.

**10.** Utilisation d'une substance immuno-suppressive, choisie parmi l'ensemble constitué par les composés de formule I et leur sels d'addition non toxiques selon la revendication 1, pour l'obtention d'un médicament destiné à une utilisation en thérapeutique vis-à-vis des désordres immunitaires.

**11.** Utilisation d'une substance choisie parmi l'ensemble constitué par les composés de formule I et leurs sels d'addition non toxiques selon la revendication 1, pour l'obtention d'un médicament destiné à une utilisation en thérapeutique vis-à-vis du paludisme.

## Claims

1. A compound belonging to the family of 15-deoxy-spergualin analogs, which is selected from the group consisting of:

   (i) the compounds of the formula

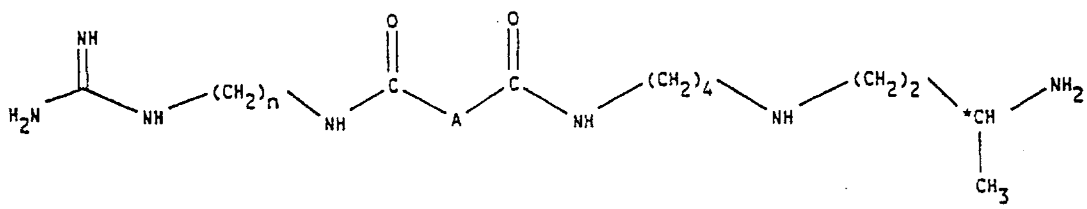

(I)

   in which:

   A is a single bond, a group $-CH_2-$, a group $-CH_2O-$ a group $-CH_2NH-$, a group $-CH(OH)-$, a group $-CHF-$ or a group $-CH(OCH_3)-$, and
   n is equal to 6 or 8; and

   (ii) their addition salts.

2. A compound according to claim 1 in which A is a group $CH_2$ or a group $CH_2O$, and its addition salts.

3. A compound according to claim 1 in which *C is a carbon having the (R) or (R,S) configuration, and its addition salts.

4. 2-[[[4-[[3-(Amino)butyl]amino]butyl]amino]carbonyloxy]-N-[6-[(aminoiminomethyl)amino]hexyl]acetamide tris(trifluoroacetate).

5. 2-[[[4-[[3(R)-(Amino)butyl]amino]butyl]amino]carbonyloxy]-N-[6-[(aminoiminomethyl)amino]hexyl]acetamide tris(trifluoroacetate).

6. A method of preparing a compound of formula I or one of its addition salts according to claim 1, said method comprising the deprotection of a compound of the formula

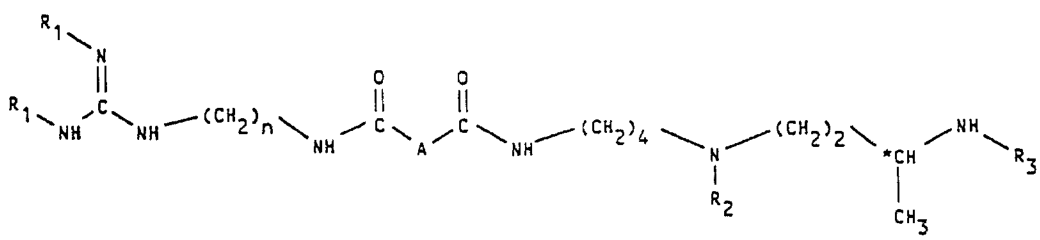

(II)

   in which:

   A is a single bond, a group $CH_2$, a group CHF, a group $CH(OCH_3)$, a group CH(OH), a group $CH(OCH_2C_6H_5)$, a group $CH_2O$ or a group $CH_2NH$,
   n is equal to 6 or 8, and
   $R_1$, $R_2$ and $R_3$, which are identical or different, are each an amino-protecting group of the alkoxycarbonyl, benzyloxycarbonyl or benzyl type,
   said deprotection comprising in particular

(α) the treatment of said compound of formula II with a strong acid when at least one of the radicals $R_1$, $R_2$ or $R_3$ is a group of the oxycarbonyl type, and/or

(β) the catalytic hydrogenation of said compound of formula II when at least one of the radicals $R_1$, $R_2$ or $R_3$ is a group of the benzyl type or when A is the group $CH(OCH_2C_6H_5)$,

to give the corresponding compound of formula I in the form of the free base or one of its addition salts.

**7.** A method according to claim 6, said method, which involves the preparation of said compound of formula II, being selected from the group consisting of:

(a) variant A, which comprises the steps consisting in:

(i) condensing a compound of the formula

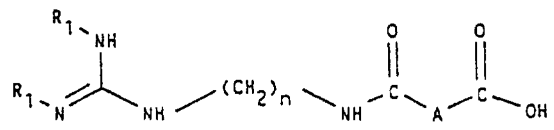

(III)

in which:

n is equal to 6 or 8,
A is the group $CH_2$, $CH(OCH_3)$, $CH(OCH_2\text{-}C_6H_5)$ or CHF or a single bond, and
$R_1$ is an amino-protecting group, with an amine of the formula

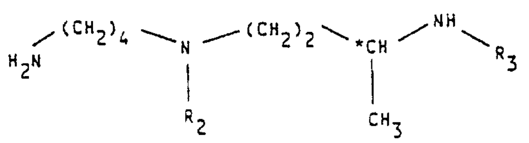

(IV)

in which:
$R_2$ and $R_3$, which are identical or different, are each an amino-protecting group,
by activation of the acid:

- either with a coupling agent of the carbodiimide type in the presence of a nucleophilic agent, in an organic solvent and at a temperature of between 0°C and 40°C,
- or by the formation of a mixed anhydride, especially with isobutyl chloroformate, in the presence of a basic agent, in an organic solvent and at a temperature of between -35°C and +20°C,

at a rate of 1 mol of the compound III to about 1 mol of the compound IV, to give a compound of the formula

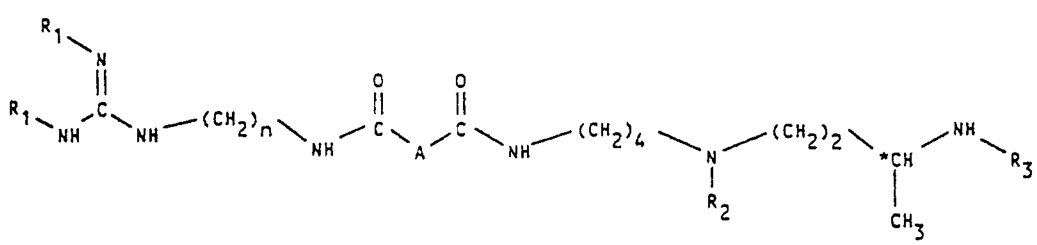

(II)

in which n, A, $R_1$, $R_2$ and $R_3$ are defined as indicated above;

(ii) deprotecting the compound of formula II obtained in step (i) by means of one or more treatments with a strong acid and/or by means of catalytic hydrogenation in order to replace $R_1$, $R_2$ and $R_3$ with a hydrogen atom to give a compound of formula I in which:

A is a single bond, $CH_2$, CHF, $CHOCH_3$ or CH(OH), and
n is equal to 6 or 8; and

(iii) if necessary, obtaining the compound of formula I in the form of the free base by reaction with a strong base, and then obtaining the other addition salts from said free base;

(b) variant B, which comprises the steps consisting in:

(i) reacting a compound of the formula

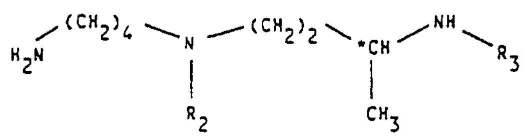

(IV)

in which:

$R_2$ and $R_3$, which are identical or different, are each an amino-protecting group of the oxycarbonyl or benzyl type,
with an acid or an acid chloride of the formula

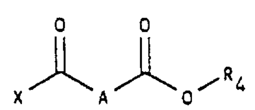

(V)

in which:
X is a chlorine atom or a group OH,
A is a single bond, a group $CH_2$, a group CHF, a group $CH(OCH_3)$ or a group $CH(OCH_2C_6H_5)$, and
$R_4$ is a linear or branched $C_1$-$C_3$-alkyl group or a phenylmethyl group,
in an organic solvent, in the presence of a carboxyl group activator and a nucleophilic agent if X is the group OH, or in the presence of a tertiary amine if X is a chlorine atom, at a temperature of between 0°C and 40°C, at a rate of 1 mol of IV to about 1 mol of V, to give a compound of the formula

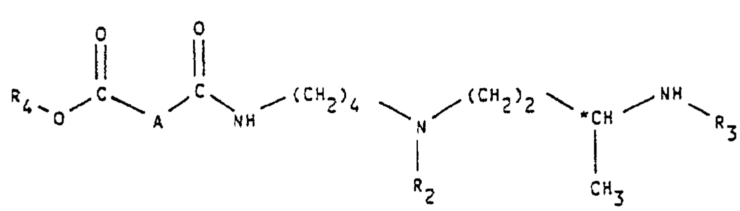

(VI)

in which A, $R_2$, $R_3$ and $R_4$ are defined as indicated above;

(ii) saponifying the resulting compound of formula VI in an organic solvent, in the presence of a strong base, to give a compound of the formula

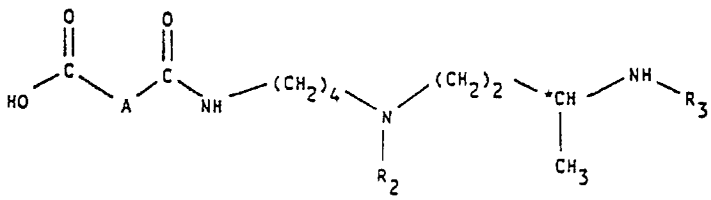

(VII)

in which A, $R_2$ and $R_3$ are defined as indicated above;

(iii) condensing the resulting compound of formula VII with an amine of the formula

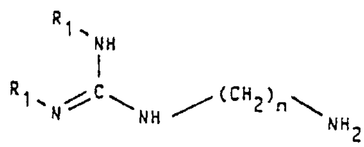

(VIII)

in which:

n is equal to 6 or 8, and
$R_1$ is an amino-protecting group,

under conditions identical to those of variant A above, to give a compound of the formula

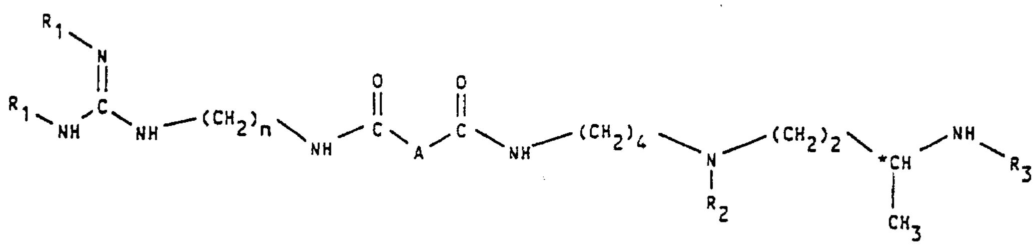

(II)

in which A, n, $R_1$, $R_2$ and $R_3$ are defined as indicated above;

(iv) deprotecting the compound of formula II obtained in step (iii) by means of one or more treatments with a strong acid and/or by means of catalytic hydrogenation in order to replace $R_1$, $R_2$ and $R_3$ with a hydrogen atom to give a compound of formula I in which:

A is a single bond, $CH_2$, CHF, $CHOCH_3$ or CH(OH), and
n is equal to 6 or 8; and

(v) if necessary, obtaining the compound of formula I in the form of the free base by reaction with a strong base, and then obtaining the other addition salts from said free base;

(c) variant C, which comprises the steps consisting in:

(i) acylating the terminal $NH_2$ end of a base of the formula

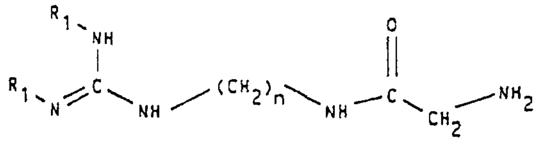

(IX)

in which $R_1$ is an amino-protecting group and n is equal to 6 or 8, with a chloroformate or a symmetrical carbonate in an inert solvent, at room temperature (15-25°C);

(ii) aminolyzing the resulting compound with an amine of the formula

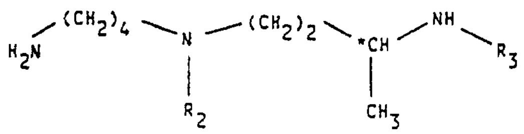

(IV)

in which $R_2$ and $R_3$, which are identical or different, are each an amino-protecting group, in an inert solvent, to give a compound of the formula

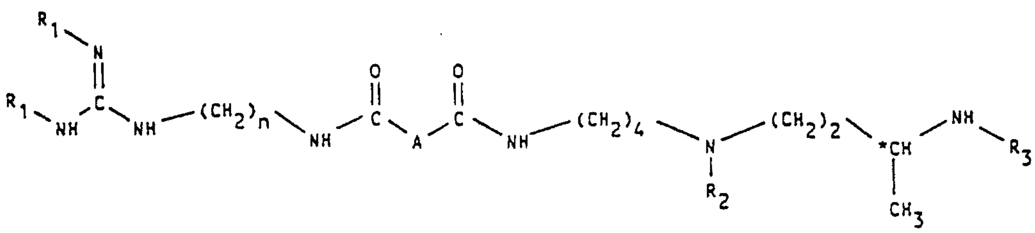

(II)

in which n, $R_1$, $R_2$ and $R_3$ are as defined above and A is the group $-CH_2NH-$;

(iii) deprotecting the compound of formula II obtained in step (ii) by means of one or more treatments with a strong acid and/or by means of catalytic hydrogenation in order to replace $R_1$, $R_2$ and $R_3$ with a hydrogen atom to give a compound of formula I in which:

A is the group $CH_2NH$, and
n is equal to 6 or 8; and

(iv) if necessary, obtaining the compound of formula I in the form of the free base by reaction with a strong base, and then obtaining the other addition salts from said free base; and

(d) variant D, which comprises the steps consisting in:

(i) reacting an amine of the formula

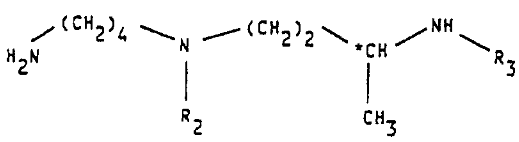

(IV)

in which $R_2$ and $R_3$, which are identical or different, are each an amino-protecting group,

with a carbonate of the formula

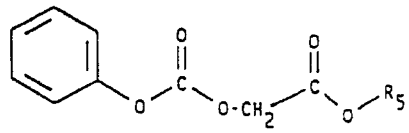

(X)

in which $R_5$ is a $C_1$-$C_3$-aikyl group or a phenylmethyl group, in an inert solvent, at a temperature between room temperature and the reflux temperature of the reaction medium, at a rate of 1 mol of IV to about 1 mol of X, to give a compound of the formula

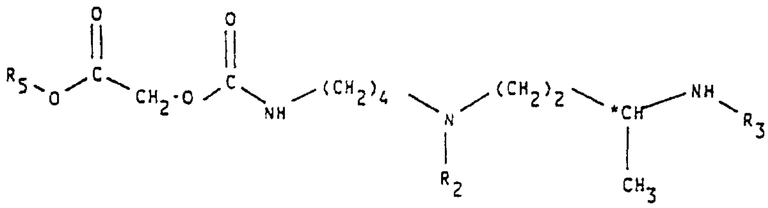

(XI)

in which $R_2$, $R_3$ and $R_5$ are as defined above, or a compound of the formula

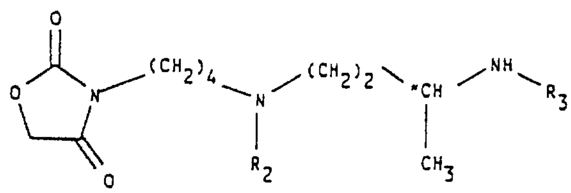

(XI')

in which $R_2$ and $R_3$ are as defined above;

(ii) saponifying the resulting compound XI or XI' in an organic solvent, in the presence of a strong base, to give a compound of the formula

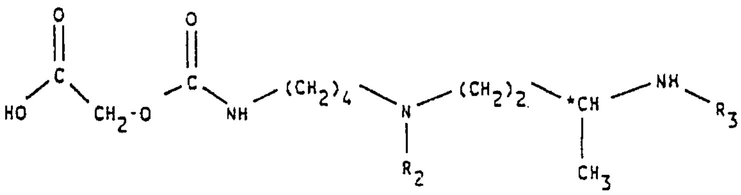

(XII)

in which $R_2$ and $R_3$ are as defined above;

(iii) condensing the resulting compound of formula XII with an amine of the formula

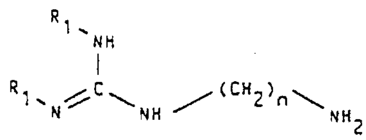

(VIII)

in which n is equal to 6 or 8 and $R_1$ is an amino-protecting group, under conditions identical to those described for variant A above, to give a compound of the formula

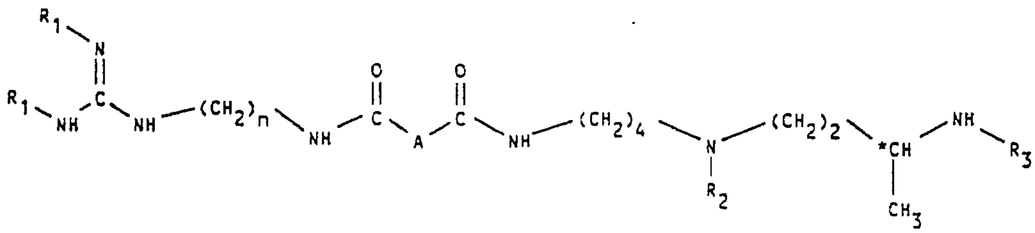

(II)

in which n, $R_1$, $R_2$ and $R_3$ are as defined above and A is a group $CH_2O$;
(iv) deprotecting the compound of formula II obtained in step (iii) by means of one or more treatments with a strong acid and/or by means of catalytic hydrogenation in order to replace $R_1$, $R_2$ and $R_3$ with a hydrogen atom to give a compound of formula I in which:

A is a group $CH_2O$, and
n is equal to 6 or 8; and

(v) if necessary, obtaining the compound of formula I in the form of the free base by reaction with a strong base, and then obtaining the other addition salts from said free base.

8. A therapeutic composition which contains, in association with a physiologically acceptable excipient, at least one compound selected from the group consisting of the compounds of formula I and their non-toxic addition salts according to claim 1.

9. An intermediate useful for the synthesis of a compound of formula I according to claim 1, which is selected from the group consisting of the compounds of the formula

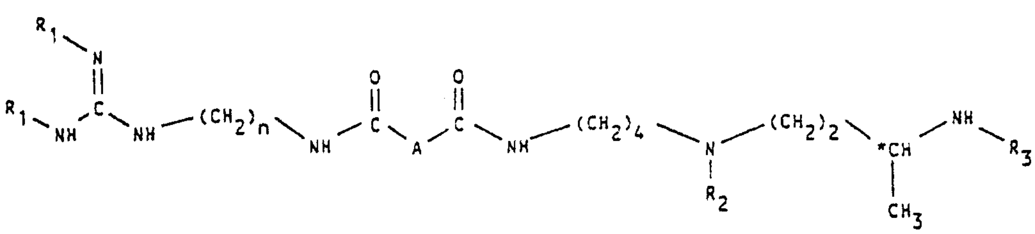

(II)

in which:

A is a single bond, a group $CH_2$, a group CHF, a group $CH(OCH_3)$, a group CH(OH), a group $CH(OCH_2C_6H_5)$, a group $CH_2O$ or a group $CH_2NH$,
n is equal to 6 or 8, and
$R_1$, $R_2$ and $R_3$, which are identical or different, are each an amino-protecting group of the alkoxycarbonyl, benzyloxycarbonyl or benzyl type.

10. A use of an immunosuppressive substance selected from the group consisting of the compounds of formula I and their non-toxic addition salts according to claim 1 for the preparation of a drug intended for use in therapeutics to combat immune disorders.

11. A use of a substance selected from the group consisting of the compounds of formula I and their non-toxic addition salts according to claim 1 for the preparation of a drug intended for use in therapeutics to combat malaria.

**Patentansprüche**

1. Verbindung, die zur Familie der Analoga von 15-Desoxyspergualin gehört, dadurch gekennzeichnet, daß sie aus der Gruppe ausgewählt ist, die aus

(i) den Verbindungen der Formel

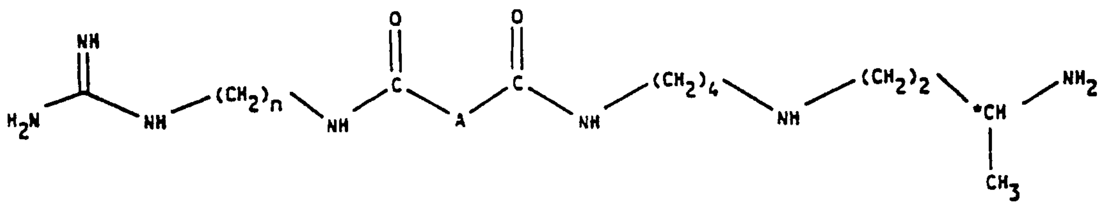

(I)

wobei

A eine Einfachbindung, eine $-CH_2$-Gruppe, eine $-CH_2O$-Gruppe, eine $-CH_2NH$-Gruppe, eine -CH(OH)-Gruppe, eine -CHF-Gruppe oder eine $-CH(OCH_3)$-Gruppe darstellt,
n gleich 6 oder 8 ist; sowie

(ii) ihren Additionssalzen
besteht.

2. Verbindung gemäß Anspruch 1, wobei A eine $-CH_2$-Gruppe oder eine $-CH_2O$-Gruppe darstellt, sowie ihre Additionssalze.

3. Verbindung gemäß Anspruch 1, wobei *C ein Kohlenstoffatom der Konfiguration (R) oder (R,S) darstellt, sowie ihre Additionssalze.

4. 2-[[[4-[[3-(Amino)butyl]amino]butyl]amino]carbonyloxy]-N-[6-[(aminoiminomethyl)amino]hexyl]acetamidtris(trifluoracetat).

5. 2-[[[4-[[3(R)-(Amino)butyl]amino]butyl]amino]carbonyloxy]-N-[6-[(aminoiminomethyl)amino]hexyl]acetamidtris(trifluoracetat).

6. Verfahren zur Herstellung einer Verbindung der Formel I oder eines ihrer Additionssalze gemäß Anspruch 1, wobei das Verfahren dadurch gekennzeichnet ist, daß es die Entfernung der Schutzgruppe von einer Verbindung der Formel

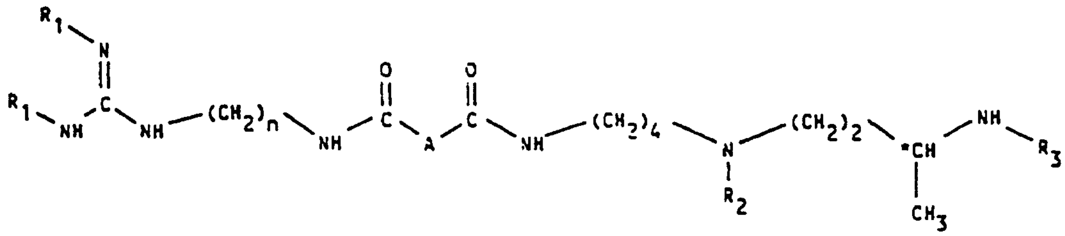

(II)

umfaßt, wobei

A eine Einfachbindung, eine $-CH_2$-Gruppe, eine -CHF-Gruppe, eine $-CH(OCH_3)$-Gruppe, eine -CH(OH)-Gruppe, eine $-CH-(OCH_2C_6H_5)$-Gruppe, eine $-CH_2O$-Gruppe oder eine $-CH_2NH$-Gruppe darstellt,
n gleich 6 oder 8 ist und
$R_1$, $R_2$, $R_3$ gleich oder verschieden sind und jeweils eine Amino-Schutzgruppe des Typs Alkyloxycarbonyl, Benzyloxycarbonyl oder Benzyl darstellen,

wobei die Entfernung der Schutzgruppe insbesondere

($\alpha$) die Behandlung der Verbindung der Formel II mit einer starken Säure, wenn wenigstens eines der Symbole $R_1$, $R_2$ oder $R_3$ eine Gruppe des Typs Oxycarbonyl darstellt, und/oder
($\beta$) die katalytische Hydrierung der Verbindung der Formel II, wenn wenigstens eines der Symbole $R_1$, $R_2$ oder $R_3$ eine Gruppe des Typs Benzyl darstellt oder wenn A die Gruppe $-CH(OCH_2C_6H_5)-$ darstellt,

beinhaltet, so daß man die entsprechende Verbindung der Formel I in Form der freien Base oder eines ihrer Additionssalze erhält.

**7.** Verfahren gemäß Anspruch 6, wobei das Verfahren, das die Gewinnung der Verbindung der Formel-II beinhaltet, dadurch gekennzeichnet ist, daß es aus der Gruppe ausgewählt ist, bestehend aus:

(a) der Variante A, die die Schritte umfaßt:

(i) Kondensieren einer Verbindung der Formel

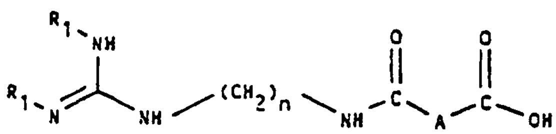

(III)

wobei

n gleich 6 oder 8 ist,
A die Gruppe $-CH_2-$, $-CH(OCH_3)-$, $-CH(OCH_2C_6H_5)-$, -CHF- oder eine Einfachbindung darstellt,
$R_1$ eine Amino-Schutzgruppe darstellt,
mit einem Amin der Formel

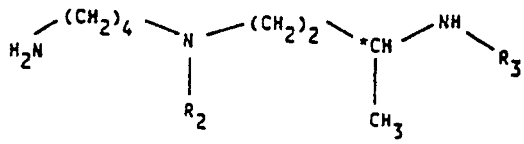

(IV)

wobei
$R_2$ und $R_3$ gleich oder verschieden sind und jeweils eine Amino-Schutzgruppe darstellen,

durch Aktivierung der Säure

- entweder mit Hilfe eines Kopplungsmittels des Carbodiimidtyps in Gegenwart eines Nucleophils in einem organischen Lösungsmittel und bei einer Temperatur zwischen 0°C und 40°C
- oder durch Bildung eines gemischten Anhydrids, insbesondere mittels Isobutylchlorformiat in Gegenwart eines basischen Mittels in einem organischen Lösungsmittel und bei einer Temperatur zwischen -35°C und +20°C im Verhältnis von 1 mol Verbindung III auf etwa 1 mol Verbindung IV, so daß man eine Verbindung der Formel

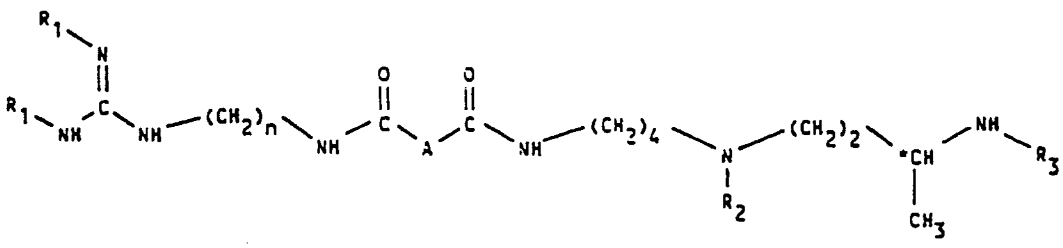

(II)

erhält, wobei n, A, $R_1$, $R_2$ und $R_3$ die oben angegebenen Bedeutungen haben;

(ii) Entfernen der Schutzgruppe von der in Schritt (i) erhaltenen Verbindung der Formel II mittels einer oder mehrerer Behandlungen mit einer starken Säure und/oder einer katalytischen Hydrierung zum Ersatz von $R_1$, $R_2$ und $R_3$ durch ein Wasserstoffatom, wobei man eine Verbindung der Formel I erhält, bei der

A eine Einfachbindung, $-CH_2-$, -CHF-, $-CH(OCH_3)-$ oder -CH(OH)- darstellt,
n gleich 6 oder 8 ist, sowie

(iii) falls notwendig, Gewinnen der Verbindung der Formel I in Form der freien Base durch Einwirkung einer starken Base, dann Gewinnen der anderen Additionssalze aus der freien Base;

(b) der Variante B, die die Schritte umfaßt:

(i) Umsetzen einer Verbindung der Formel

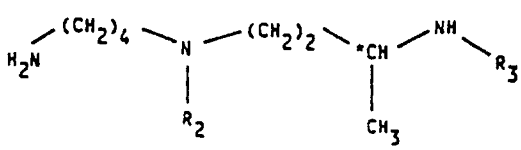

(IV)

wobei

$R_2$ und $R_3$ gleich oder verschieden sind und jeweils eine Amino-Schutzgruppe des Typs Oxycarbonyl oder Benzyl darstellen, mit einer Säure oder einem Säurechlorid der Formel

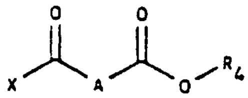

(V)

wobei

X ein Chloratom oder eine OH-Gruppe darstellt,
A eine Einfachbindung, eine $-CH_2$-Gruppe, eine -CHF-Gruppe, eine $-CH(OCH_3)$-Gruppe oder eine $-CH(OCH_2C_6H_5)$-Gruppe darstellt,
$R_4$ eine geradkettige oder verzweigte $C_1$-$C_3$-Alkylgruppe oder eine Phenylmethylgruppe darstellt, in einem organischen Lösungsmittel in Gegenwart eines Carboxygruppenaktivators und eines Nucleophils, wenn X die OH-Gruppe bedeutet, oder in Gegenwart eines tertiären Amins, wenn X ein Chloratom bedeutet, bei einer Temperatur zwischen 0°C un 40°C im Verhältnis von 1 mol IV auf etwa 1 mol V, so daß man eine Verbindung der Formel

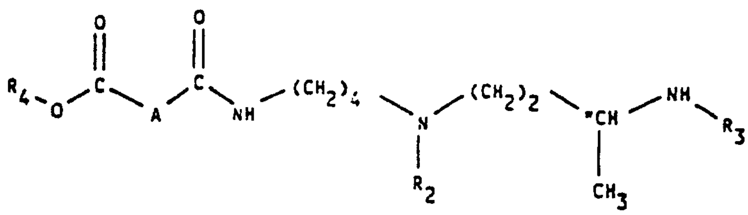

(VI)

erhält, wobei A, $R_2$, $R_3$ und $R_4$ die oben angegebenen Bedeutungen haben;

(ii) Verseifen der so erhaltenen Verbindung der Formel VI in einem organischen Lösungsmittel in Gegenwart einer starken Base, so daß man eine Verbindung der Formel

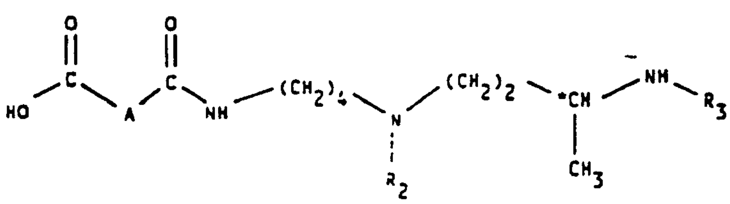

(VII)

erhält, wobei A, $R_2$ und $R_3$ die oben angegebenen Bedeutungen haben;
(iii) Kondensieren der so erhaltenen Verbindung der Formel VII mit einem Amin der Formel

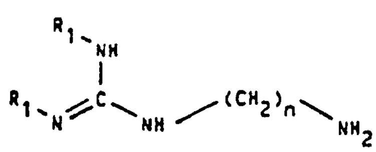

(VIII)

wobei

n gleich 6 oder 8 ist und

$R_1$ eine Amino-Schutzgruppe darstellt, unter denselben Bedingungen wie bei der obigen Variante A, so daß man eine Verbindung der Formel

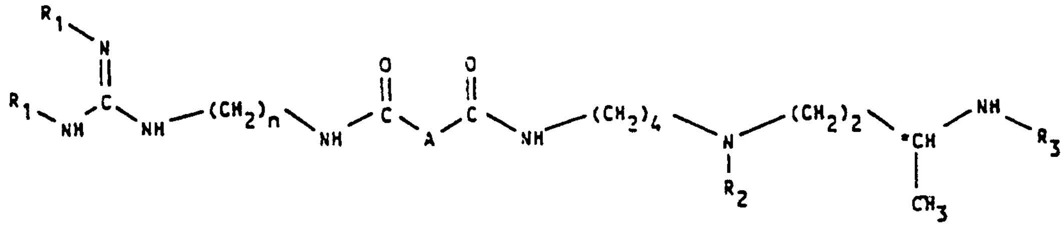

(II)

erhält, wobei A, n, $R_1$, $R_2$ und $R_3$ die oben angegebenen Bedeutungen haben;

(iv) Entfernen der Schutzgruppe von der in Schritt (iii) erhaltenen Verbindung der Formel II mittels einer oder mehrerer Behandlungen mit einer starken Säure und/oder einer katalytischen Hydrierung zum Ersatz von $R_1$, $R_2$ und $R_3$ durch ein Wasserstoffatom, wobei man eine Verbindung der Formel I erhält, bei der

A eine Einfachbindung, $-CH_2-$, -CHF-, $-CHOCH_3-$ oder -CH(OH)- darstellt und
n gleich 6 oder 8 ist, sowie

(v) falls notwendig, Gewinnen der Verbindung der Formel I in Form der freien Base durch Einwirkung einer starken Base, dann Gewinnen der anderen Additionssalze aus der freien Base;

(c) der Variante C, die die Schritte umfaßt:

(i) Acylieren des terminalen $NH_2$-Endes einer Base der Formel

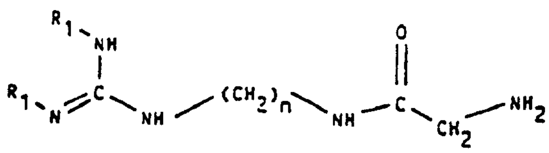

(IX)

wobei $R_1$ eine Amino-Schutzgruppe darstellt und n gleich 6 oder 8 ist,
durch ein Chlorformiat oder ein symmetrisches Carbonat in einem inerten Lösungsmittel bei Raumtemperatur (15-25°C),
(ii) Aminolysieren der so erhaltenen Verbindung durch ein Amin der Formel

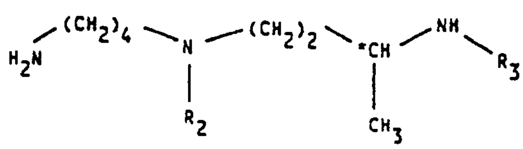

(IV)

wobei $R_2$ und $R_3$ gleich oder verschieden sind und jeweils eine Amino-Schutzgruppe darstellen, in einem inerten Lösungsmittel, so daß man eine Verbindung der Formel

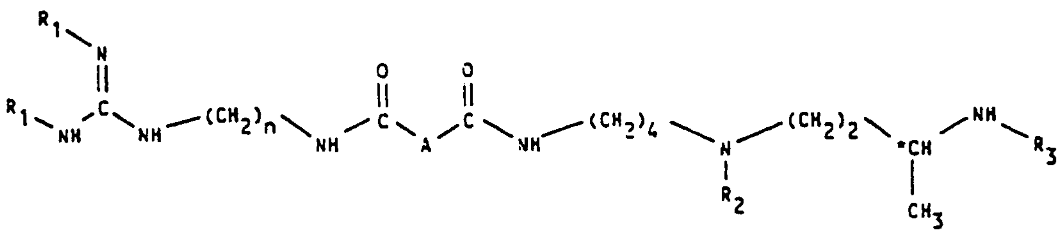

(II)

erhält, wobei n, $R_1$, $R_2$ und $R_3$ dieselben Bedeutungen wie oben haben und A die Gruppe $-CH_2NH-$ darstellt;

(iii) Entfernen der Schutzgruppe von der in Schritt (ii) erhaltenen Verbindung der Formel II mittels einer oder mehrerer Behandlungen mit einer starken Säure und/oder einer katalytischen Hydrierung zum Ersatz von $R_1$, $R_2$ und $R_3$ durch ein Wasserstoffatom, wobei man eine Verbindung der Formel I erhält, bei der A die Gruppe $-CH_2NH-$ darstellt und n gleich 6 oder 8 ist, sowie

(iv) falls notwendig, Gewinnen der Verbindung der Formel I in Form der freien Base durch Einwirkung einer starken Base, dann Gewinnen der anderen Additionssalze aus der freien Base; und

(d) der Variante D, die die Schritte umfaßt:

(i) Umsetzen eines Amins der Formel

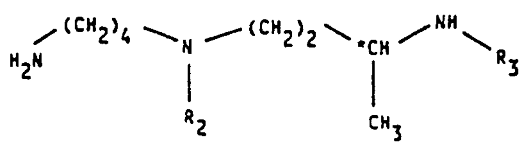

(IV)

wobei $R_2$ und $R_3$ gleich oder verschieden sind und jeweils eine Amino-Schutzgruppe darstellen, mit einem Carbonat der Formel

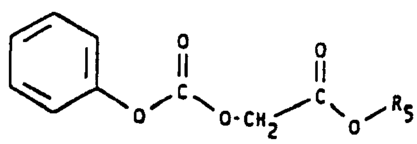

(X)

wobei $R_5$ eine $C_1$-$C_3$-Alkylgruppe oder eine Phenylmethylgruppe darstellt, in einem inerten Lösungsmittel bei einer Temperatur zwischen Raumtemperatur und der Rückflußtemperatur des Reaktionsmediums im Verhältnis von 1 mol IV auf etwa 1 mol X, so daß man eine Verbindung der Formel

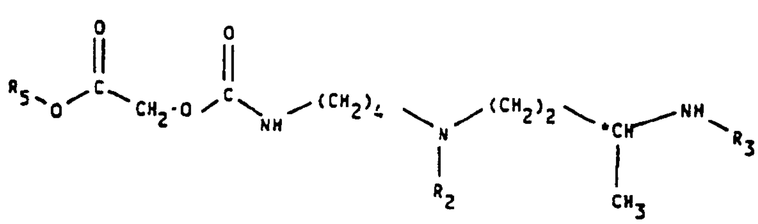

(XI)

wobei $R_2$, $R_3$ und $R_5$ dieselben Bedeutungen wie oben haben, oder eine Verbindung der Formel

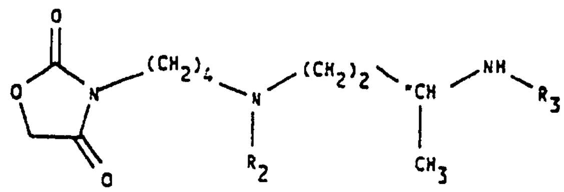

(XI')

wobei $R_2$ und $R_3$ dieselben Bedeutungen wie oben haben;

(ii) Verseifen der so erhaltenen Verbindung XI oder XI' in einem organischen Lösungsmittel in Gegenwart einer starken Base, so daß man eine Verbindung der Formel

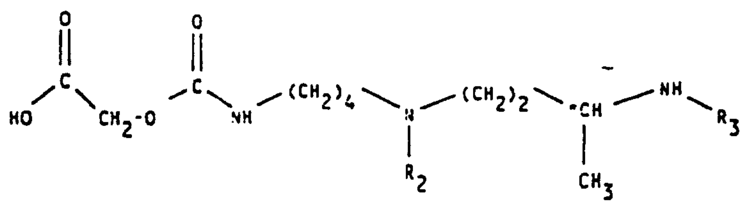

(XII)

erhält, wobei $R_2$ und $R_3$ dieselben Bedeutungen wie oben haben;

(iii) Kondensieren der so erhaltenen Verbindung der Formel XII mit einem Amin der Formel

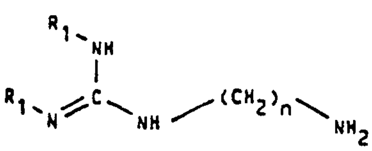

(VIII)

wobei n gleich 6 oder 8 ist und $R_1$ eine Amino-Schutzgruppe darstellt, unter denselben Bedingungen wie bei der obigen Variante A beschrieben, so daß man eine Verbindung der Formel

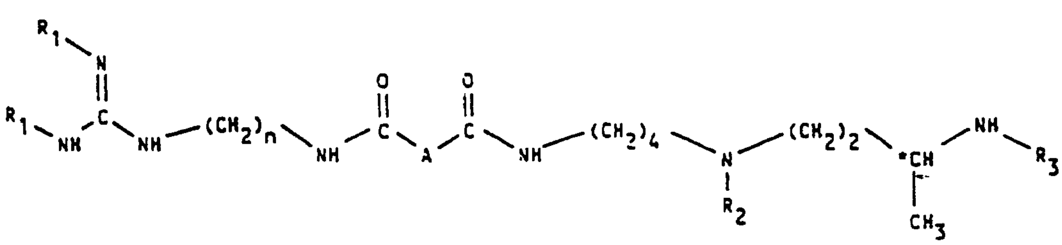

(II)

erhält, wobei n, $R_1$, $R_2$ und $R_3$ dieselben Bedeutungen wie oben haben und A eine $-CH_2O$-Gruppe darstellt;

(iv) Entfernen der Schutzgruppe von der in Schritt (iii) erhaltenen Verbindung der Formel II mittels einer oder mehrerer Behandlungen mit einer starken Säure und/oder einer katalytischen Hydrierung zum Ersatz von $R_1$, $R_2$ und $R_3$ durch ein Wasserstoffatom, wobei man eine Verbindung der Formel I erhält, bei der A eine $-CH_2O$-Gruppe darstellt und n gleich 6 oder 8 ist, sowie

(v) falls notwendig, Gewinnen der Verbindung der Formel I in Form der freien Base durch Einwirkung einer starken Base, dann Gewinnen der anderen Additionssalze aus der freien Base.

8. Therapeutische Zusammensetzung, dadurch gekennzeichnet, daß sie in Verbindung mit einem physiologisch annehmbaren Arzneimittelhilfsstoff wenigstens eine Verbindung umfaßt, die aus der Gruppe ausgewählt ist, die aus den Verbindungen der Formel I und ihren nichttoxischen Additionssalzen gemäß Anspruch 1 besteht.

9. Zwischenverbindung, die für die Synthese einer Verbindung der Formel I gemäß Anspruch 1 geeignet ist, dadurch gekennzeichnet, daß sie aus der Gruppe ausgewählt ist, die aus den Verbindungen der Formel

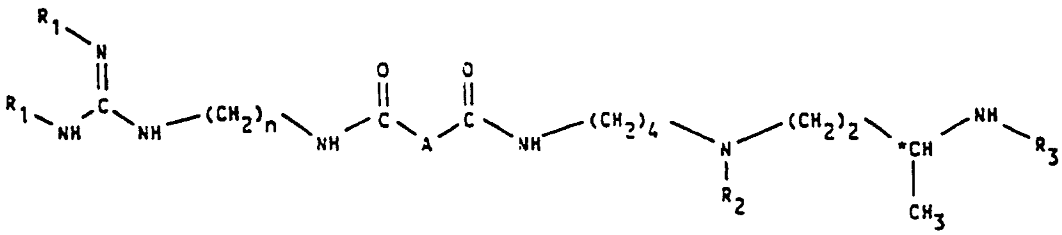

(II)

besteht, wobei

A eine Einfachbindung, eine $-CH_2$-Gruppe, eine -CHF-Gruppe, eine $-CH(OCH_3)$-Gruppe, eine -CH(OH)-Gruppe, eine $-CH-(OCH_2C_6H_5)$-Gruppe, eine $-CH_2O$-Gruppe oder eine $-CH_2NH$-Gruppe darstellt,
n gleich 6 oder 8 ist und
$R_1$, $R_2$, $R_3$ gleich oder verschieden sind und jeweils eine Amino-Schutzgruppe des Typs Alkyloxycarbonyl, Benzyloxycarbonyl oder Benzyl darstellen.

10. Verwendung einer immunosuppressiven Substanz, die aus der Gruppe ausgewählt ist, die aus den Verbindungen der Formel I und ihren nichttoxischen Additionssalzen gemäß Anspruch 1 besteht, zur Gewinnung eines Medikaments zur Verwendung bei der Therapie von Immunstörungen.

11. Verwendung einer Substanz, die aus der Gruppe ausgewählt ist, die aus den Verbindungen der Formel I und ihren nichttoxischen Additionssalzen gemäß Anspruch 1 besteht, zur Gewinnung eines Medikaments zur Verwendung bei der Therapie von Malaria.